# EUROPEAN PATENT SPECIFICATION

(11) **EP 3 427 058 B1**
(45) Date of publication and mention of the grant of the patent: **17.02.2021**
(21) Application number: 17709423.2
(22) Date of filing: 07.03.2017
(51) Int. Cl.: G01N 33/574

(54) **CHROMOGRANIN A AS A MARKER FOR BLADDER CANCER**
CHROMOGRANINE A ALS EIN MARKER FÜR BLASENKREBS
CHROMOGRANINE A COMME MARQUEUR POUR LE CANCER DE LA VESSIE

(30) Priority: 09.03.2016 EP 16159474
(43) Date of publication of application: 16.01.2019
(73) Proprietor: Cézanne S.A.S., 30035 Nimes Cédex 1 (FR)
(72) Inventor: JARDIN-WATELET, Bénédicte, 34980 Montferrier-sur-Lez (FR); BOURGOIN, Nicolas, 30620 Bernis Gard (FR); SZARVAS, Tibor, 1122 Budapest (HU)
(74) Representative: Vossius & Partner Patentanwälte Rechtsanwälte mbB
(86) International application number: PCT/EP2017/055279
(87) International publication number: WO 2017/153381

(56) References cited:
- WO-A1-2015/158701
- K A ICZKOWSKI ET AL: "Small cell carcinoma of urinary bladder is differentiated from urothelial carcinoma by chromogranin expression, absence of CD44 variant 6 expression, a unique pattern of cytokeratin expression, and more intense gamma-enolase expression", HISTOPATHOLOGY., vol. 35, no. 2, 1 August 1999 (1999-08-01) , pages 150-156, XP055290790, GB ISSN: 0309-0167, DOI: 10.1046/j.1365-2559.1999.00715.x cited in the application
- C.K. Chuang and S.K. Liao: "A retrospective immunohistochemical and clinicopathological study of small cell carcinomas of the urinary tract", Chang Gung medical journal, 1 January 2003 (2003-01-01), pages 26-33, XP055290789, Taiwan Retrieved from the Internet: URL:http://memo.cgu.edu.tw/cgmj/2601/26010 4.pdf [retrieved on 2016-07-22] cited in the application
- J.J. REINA ET AL: "Second primary malignancies in patients with neuroendocrine tumors", CLINICAL AND TRANSLATIONAL ONCOLOGY, vol. 16, no. 10, 1 May 2014 (2014-05-01), pages 921-926, XP035393124, Berlin ISSN: 1699-048X, DOI: 10.1007/S12094-014-1174-X [retrieved on 2014-05-01]
- T. HABUCHI ET AL: "Prognostic markers for bladder cancer: International Consensus Panel on bladder tumor markers", UROLOGY (ELSEVER INC), vol. 66, no. 6, 1 December 2005 (2005-12-01), pages 64-74, XP027690311, New York NK USA ISSN: 0090-4295 [retrieved on 2005-12-01]
- J.P. STEIN ET AL: "PROGNOSTIC MARKERS IN BLADDER CANCER: A CONTEMPORARY REVIEW OF THE LITERATURE", JOURNAL OF UROLOGY, LIPPINCOTT WILLIAMS & WILKINS, BALTIMORE, MD, USA, vol. 160, no. 3, 1 September 1998 (1998-09-01), pages 645-659, XP005563549, Batimore Md USA ISSN: 0022-5347, DOI: 10.1016/S0022-5347(01)62747-2
- Anonymous: "Survival rate", , Retrieved from the Internet: URL:https://en.wikipedia.org/wiki/Survival _rate [retrieved on 2016-12-08]

### SEQUENCE LISTING

<110> Cézanne S.A.S.
<120> Chromogranin A as a marker for bladder cancer
<130> Z1039 PCT BLN
<150> EP 16 15 9474.2
   <151> 2016-03-09
<160> 2
<170> BiSSAP 1.3
<210> 1
   <211> 439
   <212> PRT
   <213> Homo sapiens
<220>
   <223> Human Chromogranin A (CgA) without signal peptide
<400> 1
   Leu Pro Val Asn Ser Pro Met Asn Lys Gly Asp Thr Glu Val Met Lys
   1               5                   10                  15
   Cys Ile Val Glu Val Ile Ser Asp Thr Leu Ser Lys Pro Ser Pro Met
               20                  25                  30
   Pro Val Ser Gln Glu Cys Phe Glu Thr Leu Arg Gly Asp Glu Arg Ile
           35                  40                  45
   Leu Ser Ile Leu Arg His Gln Asn Leu Leu Lys Glu Leu Gln Asp Leu
       50                  55                  60
   Ala Leu Gln Gly Ala Lys Glu Arg Ala His Gln Gln Lys Lys His Ser
   65                  70                  75                  80
   Gly Phe Glu Asp Glu Leu Ser Glu Val Leu Glu Asn Gln Ser Ser Gln
                   85                  90                  95
   Ala Glu Leu Lys Glu Ala Val Glu Glu Pro Ser Ser Lys Asp Val Met
               100                 105                 110
   Glu Lys Arg Glu Asp Ser Lys Glu Ala Glu Lys Ser Gly Glu Ala Thr
           115                 120                 125
   Asp Gly Ala Arg Pro Gln Ala Leu Pro Glu Pro Met Gln Glu Ser Lys
       130                 135                 140
   Ala Glu Gly Asn Asn Gln Ala Pro Gly Glu Glu Glu Glu Glu Glu Glu
   145                 150                 155                 160
   Glu Ala Thr Asn Thr His Pro Pro Ala Ser Leu Pro Ser Gln Lys Tyr
                   165                 170                 175
   Pro Gly Pro Gln Ala Glu Gly Asp Ser Glu Gly Leu Ser Gln Gly Leu
               180                 185                 190
   Val Asp Arg Glu Lys Gly Leu Ser Ala Glu Pro Gly Trp Gln Ala Lys
           195                 200                 205
   Arg Glu Glu Glu Glu Glu Glu Glu Glu Glu Ala Glu Ala Gly Glu Glu
       210                 215                 220
   Ala Val Pro Glu Glu Glu Gly Pro Thr Val Val Leu Asn Pro His Pro
   225                 230                 235                 240
   Ser Leu Gly Tyr Lys Glu Ile Arg Lys Gly Glu Ser Arg Ser Glu Ala
                   245                 250                 255
   Leu Ala Val Asp Gly Ala Gly Lys Pro Gly Ala Glu Glu Ala Gln Asp
               260                 265                 270
   Pro Glu Gly Lys Gly Glu Gln Glu His Ser Gln Gln Lys Glu Glu Glu
           275                 280                 285
   Glu Glu Met Ala Val Val Pro Gln Gly Leu Phe Arg Gly Gly Lys Ser
       290                 295                 300
   Gly Glu Leu Glu Gln Glu Glu Glu Arg Leu Ser Lys Glu Trp Glu Asp
   305                 310                 315                 320
   Ser Lys Arg Trp Ser Lys Met Asp Gln Leu Ala Lys Glu Leu Thr Ala
                   325                 330                 335
   Glu Lys Arg Leu Glu Gly Gln Glu Glu Glu Glu Asp Asn Arg Asp Ser
               340                 345                 350
   Ser Met Lys Leu Ser Phe Arg Ala Arg Ala Tyr Gly Phe Arg Gly Pro
           355                 360                 365
   Gly Pro Gln Leu Arg Arg Gly Trp Arg Pro Ser Ser Arg Glu Asp Ser
       370                 375                 380
   Leu Glu Ala Gly Leu Pro Leu Gln Val Arg Gly Tyr Pro Glu Glu Lys
   385                 390                 395                 400
   Lys Glu Glu Glu Gly Ser Ala Asn Arg Arg Pro Glu Asp Gln Glu Leu
                   405                 410                 415
   Glu Ser Leu Ser Ala Ile Glu Ala Glu Leu Glu Lys Val Ala His Gln
               420                 425                 430
   Leu Gln Ala Leu Arg Arg Gly
           435
<210> 2
   <211> 267
   <212> PRT
   <213> Homo sapiens
<220>
   <223> Human matrix metalloproteinase 7 (MMP7)
<400> 2
   Met Arg Leu Thr Val Leu Cys Ala Val Cys Leu Leu Pro Gly Ser Leu
   1               5                   10                  15
   Ala Leu Pro Leu Pro Gln Glu Ala Gly Gly Met Ser Glu Leu Gln Trp
               20                  25                  30
   Glu Gln Ala Gln Asp Tyr Leu Lys Arg Phe Tyr Leu Tyr Asp Ser Glu
           35                  40                  45
   Thr Lys Asn Ala Asn Ser Leu Glu Ala Lys Leu Lys Glu Met Gln Lys
       50                  55                  60
   Phe Phe Gly Leu Pro Ile Thr Gly Met Leu Asn Ser Arg Val Ile Glu
   65                  70                  75                  80
   Ile Met Gln Lys Pro Arg Cys Gly Val Pro Asp Val Ala Glu Tyr Ser
                   85                  90                  95
   Leu Phe Pro Asn Ser Pro Lys Trp Thr Ser Lys Val Val Thr Tyr Arg
               100                 105                 110
   Ile Val Ser Tyr Thr Arg Asp Leu Pro His Ile Thr Val Asp Arg Leu
           115                 120                 125
   Val Ser Lys Ala Leu Asn Met Trp Gly Lys Glu Ile Pro Leu His Phe
       130                 135                 140
   Arg Lys Val Val Trp Gly Thr Ala Asp Ile Met Ile Gly Phe Ala Arg
   145                 150                 155                 160
   Gly Ala His Gly Asp Ser Tyr Pro Phe Asp Gly Pro Gly Asn Thr Leu
                   165                 170                 175
   Ala His Ala Phe Ala Pro Gly Thr Gly Leu Gly Gly Asp Ala His Phe
               180                 185                 190
   Asp Glu Asp Glu Arg Trp Thr Asp Gly Ser Ser Leu Gly Ile Asn Phe
           195                 200                 205
   Leu Tyr Ala Ala Thr His Glu Leu Gly His Ser Leu Gly Met Gly His
       210                 215                 220
   Ser Ser Asp Pro Asn Ala Val Met Tyr Pro Thr Tyr Gly Asn Gly Asp
   225                 230                 235                 240
   Pro Gln Asn Phe Lys Leu Ser Gln Asp Asp Ile Lys Gly Ile Gln Lys
                   245                 250                 255
   Leu Tyr Gly Lys Arg Ser Asn Ser Arg Lys Lys
               260                 265

## Description

### Field

The present invention is in the field of clinical diagnostics. Particularly, the invention relates to the prognosis, risk assessment, risk stratification, monitoring and/or therapy control of non-neuroendocrine bladder cancer based on chromogranin A (CgA) as a marker.

### Background

Bladder cancer (urinary bladder cancer) is the most common malignancy of the urinary tract. Considering newly diagnosed cases and its high recurrence rate, bladder cancer is one of the most prevalent cancers worldwide (Chamie et al. 2011, Goodison, Rosser and Urquidi 2013). Western countries are more affected and men have 3-4 fold higher risks to develop as compared to women (Burger et al. 2013a).
Based on the cell that becomes cancerous, different types of bladder cancer can be distinguished. The most common type of bladder cancer is transitional cell carcinoma (90%) followed by squamous cell carcinoma and adenocarcinoma (2-5%). Less than 1% of bladder cancers arise from neuroendocrine cells (Bertaccini et al. 2008, Pompas-Veganzones, Gonzalez-Peramato and Sanchez-Carbayo 2014). At diagnosis about 70-75% of patients have superficial bladder cancer (stages Ta-T1) which is also known as non-muscle invasive bladder cancer (NMIBC). 25-30% of patients have muscle invasive bladder cancer (MIBC, stages T2-T4) and/or metastatic bladder cancer (Clark et al. 2013, Mossanen and Gore 2014).
The recurrence rate and or progression of bladder cancer is dramatically high with 50-70% of patients with NMIBC that will have disease recurrence, progression or a new occurrence within 5-7 years following treatment (Clark et al. 2013) and 10% to 30% of these recurrences are invasive (Chamie et al. 2011, Goodison et al. 2013, Witjes et al. 2014). Approximately half (43%) of the MIBC cases had non-invasive tumors at diagnosis, that progressed despite organ-preserving treatment (EAU Guidelines on Muscle-invasive and Metastatic Bladder Cancer 2014).

Currently, the cornerstone of diagnosis and surveillance of bladder cancer is the histopathological examination of biopsies from the bladder urothelium during cystoscopy / Trans-Urethral Resection of Bladder Tumor (TURBT). White light cystoscopy is a useful diagnostic tool in the hand of clinicians but suffers major limitations. Indeed it frequently understages tumors which may lead to insufficient treatment (Cauberg Evelyne, de la Rosette and de Reijke 2011). It is also moderately sensitive, expensive and invasive. Some extremely rare complications as obturator reflex perforation, bleeding, TUR Syndrome, urinary tract obstruction, perforation (extra or intra-peritoneal) and infections may occur during this procedure.
Voided Urine Cytology (VUC) is also a widely used method for the diagnosis and surveillance of NMIBC with high specificity but lower sensitivity in well differentiated tumors (low grade). Its accuracy ranging from only 20 to 40 % for early stage tumors (AUA Guidelines 2007-2014, Goodison et al., 2013). It showed better accuracy for high grade tumors and particularly for carcinoma in situ. This feature makes it a complementary tool for transurethral resection (see below). Cystoscopy and VUC allow a first diagnosis and a first evaluation of tumor features, including number, type, grade and estimated stage.
Computed tomography (CT) and magnetic resonance imaging (MRI) may also be used for primary tumor evaluation. However, in up to 40% of cases, it underestimates the disease and can only marginally differentiate between tumor stages Ta to T3a (accuracy rates range from 55-92%) even if the accuracy differentiating invasive from non-invasive is reported as better (Maurer et al. 2013).
The evaluation of metastatic status, and especially nodal status, is a key point of prognostic evaluation of bladder cancer. Lymph node metastasis increases from a low rate of 5-10% in non-muscle-invasive bladder tumors to 15-20% in superficial muscle-invasive tumors, to 25-30% in deep muscle-invasive tumors and to >40% in extravesical tumors (Shariat et al. 2012). Diagnosis of distant or regional metastatic bladder cancer is largely based on a variety of imaging techniques including radiography, ultrasonography, computed tomography (CT) and magnetic resonance imaging (MRI). Ultrasonography is the simplest, most non-invasive, and cost-effective, but relies on the skill of the operator. CT and MRI are also non- invasive, and although the sensitivities of these techniques have recently been improved, its performances remain insufficient.
In case the first diagnosis is positive for bladder cancer regardless of metastatic status, a Trans-Urethral Resection of Bladder Tumor (TURBT) is performed. This theranostic method is the initial therapeutic step characterized by the endoscopic ablation of the whole tumor. It confirms the diagnosis and authorizes a more precise evaluation of tumor stage, especially the depth of bladder wall invasion. However, TURBT is associated with a significant risk of understaging, especially for T1 tumors (Babjuk 2009).
Moreover, based on incomplete resection, recurrent tumor growth can be observed during the first year (Kamat et al. 2013, Babjuk 2009). For this reason repeated TURBT or maximal TURBT is highly recommended by guidelines (Brausi et al. 2011).

While patients with NMIBC can often be safely managed with "perfect" TURBTs with or without additional therapeutic measures (e.g. immunotherapy, chemotherapy), radical cystectomy (RCE) with bilateral pelvic lymph-node dissection (PLND) has become the standard of care for patients with MIBC (Clark et al. 2013, Witjes et al. 2014). Some neoadjuvant chemotherapies have shown survival benefit (Sharma, Ksheersagar and Sharma 2009) and are recommended for selected patients. RCE is also recommended for high grade and/or refractory NMIBC and Carcinoma In Situ (CIS). Many patients with radical surgery undergo urinary diversion or neo-bladder reconstruction. National Cancer Comprehensive Network (NCCN) guidelines recommend that node negative patient with highest pathological estimated risk may receive adjuvant additional radiotherapy with radiosensitizing therapy or chemotherapy. However, these additional therapies may improve toxicity and comorbidity (Clark et al. 2013).
Following RCE alone in overall bladder cancer (BCa) (all stage and grade), the 5-year overall, recurrence-free, and cancer-specific survival rates were only 57%, 48%, and 67%, respectively, with distant and local recurrence rates of 37% and 6%, respectively (Yafi et al. 2011).
The benefits on patient survival of standard pelvic lymphe-node dissection (PLND) for MIBC cases, have been largely demonstrated and PLND is today an integral part of RC consensually recommended by guidelines. However, its benefit in a surgical monotherapy strategy is moderate and lymphadenectomy (LND) is often considered as more diagnostic / prognostic (may supply nodal status) than therapeutic (Skinner and Sagalowsky 2014). Indeed, regional LND is a necessary step in staging due to the limitations in sensitivities of current imaging techniques. Despite advancements in surgical technique, imaging, perioperative management and therapies, approximately 50% of patients develop metastases and die from bladder cancer (Stein JP, Skinner DG 2006).
Then, neoadjuvant/adjuvant chemotherapies or radiotherapy and PLND or extended LND are considered (Skinner and Sagalowsky 2014) to improve the survival of high risk patients. However, due to increase of toxicity, risk of comorbidity and cost, the benefits of these heavy interventions must be perfectly balanced against quality of life changes and potential complications, including bleeding, nerve injury, lymphocele or -extremity thrombus (Scarpato et al. 2015) and dedicated to the highest risks cases.
One of the challenges of this kind of advanced malignancies is to answer the question: Which treatment for which patient?

Following RCE with or without LND, Helical CT represents the imaging modality of choice to identify lung, lymph node, and liver metastasis (ICUD-EAU Bladder cancer Edition 2012) and EAU guidelines recommend 3-4-monthly of abdomen, Upper Urinary Tract (UUT), Pelvis scans and chest radiography or CT (Sharma et al. 2009) during the first year, 6-monthly until the third year and after this period monitoring by annual imaging as clinically indicated. For RCE with cutaneous Urinary Diversion (UD) urethral wash cytology every six to 12 months is also recommended (Sharma et al. 2009). Frequency and methods for follow-up post radical cystectomy are not strictly consensual between guidelines, however only slightly different.

Distant recurrence occurs usually within 24 months, with higher stage and lymph node status as the most important risk factors. Pelvic recurrence typically occurs (5-15% of patients) in 6-18 months after surgery, depending again on the initial stage and lymph node status. Secondary urethral tumours are rare, occurring 1-3 years after cystectomy and have poor survival rate. Upper urinary tract recurrence is rarely seen and usually presents late (28-49 months after cystectomy) (Witjes et al. 2014). Finally, some late recurrences may occur after more than 10 years then following RCE, patients need also a very long-term surveillance.

Due to the prognostic heterogeneity of both NMIBC and MIBC, guidelines for bladder cancer underline the importance and unmet needs for better prognostication (EAU guidelines). For NMIBC risk tables are available to improve decisions on treatment and/or follow-up (Vedder et al. 2014). The European Organization for Research and Treatment of Cancer (EORTC) scoring system recommended by EAU and AUA guidelines is however rarely used in daily routine.
It has been shown in a number of recent publications that these models were not firmly reliable. For example, Vedder et al. published that the scoring system could reasonably predict progression but not recurrence (Vedder et al. 2014).
Recurrence and progression risk overestimation by EORTC table has also been demonstrated in NMIBC, also in BCG-treated subgroup (Fernandez-Gomez et al., 2011). Finally, Xylinas et al. demonstrated that the EORTC scoring system show a poor discrimination for both recurrence and progression and underlined the need for improving the current predictive tools (Xylinas et al. 2013).
To date, there is no table or scoring system to specifically evaluate the prognosis of MIBC so to guide the treatment choice. The presence of metastases in regional lymph node is the strongest predictor of tumor recurrence and disease-specific survival (DSS) in patients for MIBC (Skinner and Sagalowsky 2014). However approximately half of superficial tumors may have micrometastases undetected by imaging tools, while only 25% of radical surgery patients have lymph node (LN) metastases at the time of surgery (Svatek et al. 2010), EAU Guidelines on Muscle-invasive and Metastatic Bladder Cancer 2014).
Even though disease specific survival (DSS) ranges from 85% to 90% following radical cystectomy for CIS, early radical cystectomy can be considered as overtreatment in approximately 50% of patients (Burger et al. 2013b).

Bladder cancer is a heterogeneous neoplasm that needs a large scale of treatment algorithm (sequential chemical or biological treatment, radiotherapy and surgical interventions) and an extensive long-term surveillance. Tumors with similar histology may have different clinical behaviors and these nuances are critical to proper management. Its complexity results in direct and indirect economic burden and makes it the most expensive malignancy in terms of lifetime medical care costs on per-patient basis (Brausi 2013, Chamie et al. 2011, Goodison et al. 2013, Mossanen and Gore 2014). In 2010, the medical costs for BCa was approximately $4 billion in the USA (Mossanen and Gore 2014) and €3 billion in 2012 in the European Union (EU) including direct inpatient care as being the major cost component (accounting for 58%). The global economic burden was €5 billion in the EU (Leal et al. 2015). In the USA, the estimated cost for lifetime individual management of BCa is ranging from $96,000 to $280,000 (Hansel et al. 2013). The medical costs associated with a MIBC diagnosis is approximately $150,000, however due the protracted clinical course of early stage disease, its prevalence relatively to MIBC and its procedure-oriented surveillance, the economic burden of NMIBC is generally considered to be more elevated (Svatek et al. 2014).
Tumor understaging is the main limitation of the current diagnosis and prognostication tools. Moreover, to date, there is no reliable tool to predict the progression of NMIBC to MIBC neither to predict the outcome of MIBC.

The use of biomarkers for prognosis or risk stratification of bladder cancer patients would help the physician to initiate a suitable treatment and to reduce costs associated with patient surveillance.
Some commercial urinary markers have received FDA approval for the diagnosis and/or follow-up of bladder cancer but did not penetrate the clinical practice. In contrast, currently no blood or urinary prognosis markers have been approved by national health institutions or recommended by any guidelines. This is not coherent with the strategy of global risk-based management of BCA.

In a recent large meta-analysis (Schmitz-Drager et al. 2015), the performances of the most commonly used commercially available markers (BTA Stat ®, NMP22 including BladderCheck ®, and FISH Urovysion ™) have been compared to VUC. It confirms that these molecular markers have a largely better sensitivity, especially for high risk tumors (pT1G3, CIS) than urine cytology despite no demonstrated prognosis application. However, it also confirms that the lower specificity and reproducibility of such markers was a major limitation. In addition, these markers had no prognostic value.

Chromogranin A (CgA) is a glycoprotein commonly expressed in neuroendocrine (NE) cells. CgA is a constituent of the secretory granules of most peptide-producing endocrine cells (Chuang and Liao 2003). It is physiologically released by exocytosis and can be detected in blood. When a tumor develops in a neuroendocrine tissue, it becomes the main source of circulating CgA. CgA secretion in the urinary bladder is associated with the rare cases of neuroendocrine differentiated tumors which are known to have a poor prognosis (Alijo Serrano et al. 2007, Bertaccini et al. 2008). Neuroendocrine tissue markers, for example CgA, are frequently used for differentiation between neuroendocrine carcinoma (NEC) and Transitional Cell Carcinoma (TCC) and used to confirm diagnosis of NEC and typically for small cell bladder cancer (SCBC) (Bertaccini et al. 2008, Cerulli et al. 2012, Iczkowski et al. 1999) or paragangliomas (Bagchi et al. 2015, Feng et al. 2013).

WO 2015/158701 A1 relates to immunoassay and antibodies for the detection of chromogranin A. Iczkowski et al., (Histopathology vol. 35, no. 2, p. 150-156 (1999)) discloses that small cell carcinoma of urinary bladder is differentiated from urothelial carcinoma by chromogranin expression, absence of CD44 variant 6 expression, a unique patter of cytokerating expression, and more intense γ-enolase expression. Chuang and Liao (Chang Gung Medical Journal 2003, p. 26-33) discloses retrospective immunohistochemical and clinicopathological study of small cell carcinomas of the urinary tract. Reina et al., (Clinical and Translational Oncology, vol. 16, no. 10 (2014), pages 921-926) relates to second primary malignancies in patients with neuroendocrine tumors. Habuchi et al., (Urology, vol. 66, no. 6 (2005), p. 64-74) relates to prognostic markers for bladder cancer: International Consensus Panel on bladder tumor markers. Stein et al., (Journal of Urology, vol. 160, no. 3 (1998), p. 645-659) discloses prognostic markers in bladder cancer: a contemporary review of the literature.

However, CgA has been demonstrated to be expressed in tissue of neuroendocrine bladder cancer (NEBC), but to date, no study assessed the prognostic effect of tissue and serum CgA levels neither in neuroendocrine nor in urothelial BCAs.

Moreover, the presence of both small cells and transitional cells did not predict outcomes either (Chuang and Liao 2003).

Chuang et al., have shown that CgA was expressed in 4 of 10 SCBC tissue samples but found no association with patients' prognosis. However due to the low number cases and heterogeneous cohort, this study has limited reliability (Chuang and Liao 2003).

CgA did not show any relationship with survival in large cell and small cell neuroendocrine bladder carcinoma. In one study, the only prognostic factor for survival remained the TNM classification (Alijo-Serrano, 2007). The authors did not consider CgA as useful for prognostic purposes. Moreover Soukup et al., recently found that urine CgA was neither related to the presence of primary NMIBC nor to cancer recurrence (Soukup et al. 2015). Nevertheless, Bertaccini et al., have found that 1 month after cystoprostatectomy for Small Cell Urothelial Carcinoma (SCUC), the serum level of CgA decreased 10-fold compared to preoperative level but did not assessed preoperative prognosis neither follow-up value (Bertaccini et al. 2008).

Matrix metalloproteinase-7 (MMP7) has been described as an independent marker of lymph nodes metastases in serum and urine (Gunes et al. 2013, Jager et al. 2013, Szarvas et al. 2010, Szarvas et al. 2011b) and also as an independent prognostic marker for BCa pre-surgery (Svatek et al. 2010, Szarvas et al. 2010, Szarvas et al. 2011a). High MMP-7 levels have also been described as an independent risk factor of poor survival in specific metastatic bladder cancers (E1 Demery et al. 2014). Also see WO 2007/144144 A1.

The present invention is based on the surprising finding that Chromogranin A has a high prognostic value in non-neuroendocrine bladder cancer. As such it can help overcome the above shortcomings of the prior art diagnostic tools for bladder cancer.

### Summary

The invention relates to the embodiments as defined in the claims. The present invention relates to the in vitro use of Chromogranin A (CgA) as a marker (particularly a prognostic marker) for non-neuroendocrine bladder cancer and preferably urothelial carcinoma. In particular, CgA can be used as a marker in an in vitro assay for the prognosis, risk assessment, risk stratification, monitoring and/or therapy control of non-neuroendocrine bladder cancer and preferably urothelial carcinoma.

The invention further pertains to an in vitro method for the prognosis, risk assessment, risk stratification, monitoring and/or therapy control of non-neuroendocrine bladder cancer and preferably urothelial carcinoma, in a subject, comprising the step of determining the level of CgA and optionally MMP7 in a sample of a bodily fluid of said subject. The present invention further relates to the in vitro use of diagnostic kits comprising one or more antibodies specific for CgA for the prognosis, risk assessment, risk stratification, monitoring and/or therapy control of non-neuroendocrine bladder cancer and preferably urothelial carcinoma, in a subject. The disclosure also pertains to methods of treating bladder cancer, particularly non-neuroendocrine bladder cancer and preferably urothelial carcinoma, in a subject, wherein the level of CgA in a sample from said subject is determined.

### Description of the drawings

- Figure 1:: Serum concentration of CgA in controls and cases
- Figure 2:: Prognostic value of CgA and MMP-7 levels and their combination in surgically treated (TURBT or RCE) patients (Kaplan-Meier curves with log rank tests). DSS: disease specific survival.
- Figure 3:: Prognostic value of CgA and MMP7 levels and their combinations in the subgroup of RCE treated patients (Kaplan-Meier curves with log rank tests)

### Detailed description

The present invention relates to the in vitro use of Chromogranin A (CgA) as a marker for non-neuroendocrine bladder cancer, and preferably urothelial carcinoma, particularly as a prognostic marker for for non-neuroendocrine bladder cancer, preferably urothelial carcinoma. Hence, CgA can be used as a marker in an in vitro assay for the prognosis, risk assessment, risk stratification, monitoring and/or therapy control of non-neuroendocrine bladder cancer, preferably urothelial carcinoma. In other words, CgA has turned out to be a good biomarker for the assessment of disease severity in non-neuroendocrine bladder cancer patients, preferably in urothelial carcinoma patients. CgA may, thus, also be used in the management of patients ("patient management") with non-neuroendocrine bladder cancer and preferably urothelial carcinoma.

The present invention relates to in vitro methods for the prognosis of subjects with with non-neuroendocrine bladder cancer, preferably subjects with urothelial carcinoma, comprising the step of determining the level of CgA (and optionally MMP7) in a sample of a bodily fluid of said subject. The invention in particular pertains to an in vitro method for the prognosis, risk assessment, risk stratification, monitoring and/or therapy control of non-neuroendocrine bladder cancer, preferably urothelial carcinoma, in a subject, comprising the step of determining the level of CgA in a sample of a bodily fluid of said subject. Optionally the level of MMP7 is additionally determined in the same or another sample of said subject. The level of CgA (and optionally MMP7 as the case may be) in the sample from the subject is in one aspect indicative for the severity and aggressiveness of non-neuroendocrine bladder cancer, preferably urothelial carcinoma, and/or the outcome for the subject. An increased level of CgA (and optionally MMP7 as the case may be) in the sample from the subject as compared to a control level or a predetermined threshold is indicative for a poor outcome for the subject. For example, the level of CgA in the sample from the subject is indicative for the subject's overall survival or the subject's disease-specific survival or the subject's progression-free survival.

In some embodiments first the level of CgA is determined in a sample of the subject and subsequently further tests are performed if the CgA level is above the pre-determined threshold in another sample of the same subject, e.g. subsequently the level of MMP7 is determined in order to refine or confirm the initial prognosis based on the CgA level. In other, more preferred, embodiments first the level of MMP7 is determined in a sample of the subject and subsequently further tests are performed if the MMP7 level is above the pre-determined threshold in another sample of the same subject, e.g. subsequently the level of CgA is determined in order to refine or confirm the initial prognosis based on the MMP7 level. Hence, MMP7 and CgA levels can be used together in order to refine the initial prognosis based on only one of these two markers.

The term "bladder cancer" (also referred to as "urinary bladder cancer") in the context of the present invention relates to bladder cancer of non-neuroendocrine origin. Hence, preferably the "bladder cancer" is not a neuroendocrine tumor (NET). The term "neuroendocrine" pertains to neural or endocrine influence and particularly to the interaction between the nervous and endocrine system. In particular, the term "neuroendocrine" relates to the cells that release a hormone into the blood in response to a neural stimulus. "Neuroendocrine cancers" consequently are malignant neoplasms that arise from cells of the endocrine (hormonal) and nervous systems. Neuroendocrine bladder cancer includes for example small cell carcinoma (SCC), carcinoid tumor and large cell neuroendocrine carcinoma (LCNEC).
Hence, "bladder cancer" herein preferably refers to a bladder cancer which does not arise from cells of the endocrine (hormonal) and nervous systems. Therefore, preferably herein, bladder cancer is not a small cell carcinoma, not a carcinoid tumor and not a large cell neuroendocrine carcinoma. The bladder cancer in the context of the present invention is preferably selected from the group consisting of transitional cell carcinoma (TCC) (i.e. urothelial carcinoma (UC) of the bladder, also known as urothelial cell carcinoma (UCC)), squamous cell carcinoma and adenocarcinoma, more preferably the bladder cancer in the context of the present invention is a transitional cell carcinoma. The term "urothelial" specifically refers to a carcinoma of the urothelium, meaning a TCC of the urinary system. Hence, urothelial carcinoma in the context of the present invention means urothelial carcinoma of the bladder. The transitional cell carcinoma (= urothelial carcinoma) can be a superficial bladder cancer (i.e. non- muscle invasive bladder cancer (NMIBC)) or a muscle invasive bladder cancer (MIBC). The NMIBC can, e.g., be a papillary carcinoma or a flat carcinoma (e.g. a carcinoma in situ (CIS)). In general, urothelial carcinoma of the bladder can, e.g., also be classified as micropapillary, nested, plasmocytoid, sarcomatoid or other variants or can have mixed histologies. The micropapillary or nested variants of urothelial carcinoma are particularly aggressive. These bladder cancers may, for instance, be classified according to UICC/AJCC (Union for international cancer control / American Joint Committee on Cancer) TNM table and graded according to WHO 1973-2004 classifications.

The subject in the context of the present invention suffers from and/or has been diagnosed with non-neuroendocrine bladder cancer prior to said determination of the CgA level. Preferably, herein, the subject does not have (i.e. suffer from) a neuroendocrine bladder cancer. Hence, preferably the subject has not been diagnosed with a neuroendocrine bladder cancer.

The term "subject" herein refers to a living human or non-human animal, preferably a mammal, most preferably a human. The subject is preferably a patient. The term "patient" as used herein refers to a living human or non-human animal (most preferably a human) that is receiving medical care or that should receive medical care due to a disease, particularly non-neuroendocrine bladder cancer. This includes persons with no defined illness who are being investigated for signs of pathology. Thus the methods and assays described herein are applicable to both, human and veterinary disease.

The term "level" in the context of the present invention relates to the concentration (preferably expressed as weight/volume; w/v; e.g. as "ng/mL") of a marker (e.g. CgA and/or MMP7) taken from a sample of a subject, e.g. a bladder cancer patient.

"Diagnosis" in the context of the present invention relates to the recognition and (early) detection of a disease or clinical condition in a subject and may also comprise differential diagnosis. Also the assessment of the severity of a disease or clinical or histopathological condition may in certain embodiments be encompassed by the term "diagnosis".
"Prognosis" relates to the prediction of an outcome or a specific risk for a subject suffering from a particular disease or clinical condition, here non-neuroendocrine bladder cancer. This may include an estimation of the chance of recovery or the chance of an adverse outcome for said subject.

"Monitoring" or "therapy monitoring" relates to keeping track of an already diagnosed disease, disorder, complication or risk, e.g. to analyze the progression of the disease (here: non-neuroendocrine bladder cancer) or the influence of a particular treatment on the progression of disease or disorder. In the present invention, the term "risk stratification" relates to the grouping of subjects into different risk groups according to their further prognosis. Risk stratification also relates to stratification for applying preventive and/or therapeutic measures.

The term "patient management" in the context of the present invention refers to:
- the decision for admission to hospital or intensive care unit,
- the decision for relocation of the patient to a specialized hospital or a specialized hospital unit,
- the evaluation for an early discharge from the intensive care unit or hospital,
- the allocation of resources (e.g. physician and/ or nursing staff, diagnostics, therapeutics, surgery).

The term "assessment of disease severity" relates to the evaluation of the status of the disease in a patient including the histopathology of the tumor, the metastatic status and progression of the disease, the likelihood of adverse events (including death), the likelihood of high inpatient expenses and the likelihood of lengthy hospital stay.

The term "outcome" in the context of the present invention relates to the degree of severity of the disease, e.g. the state of health of a patient after a defined time, e.g. after 3 days, 5 days, 10 days, 14 days, 20 days, 3 weeks, 4 weeks, 30 days, 45 days, 60 days, 90 days, 3 months, 6 months, 1 year, 2 years, or 5 years. The outcome can be expressed as a rate (percentage) in a population (e.g. study or treatment group) or as an individual rate (i.e. probability for a specific subject or patient). Hence the "overall outcome" can be expressed as an "overall survival rate" which is the percentage of people in a group (e.g. study or treatment group) who are still alive for a certain period of time after they were diagnosed with or started treatment for a disease, such as in the present case non-neuroendocrine bladder cancer. The overall survival rate is often stated as a five-year survival rate, which is the percentage of people in a study or treatment group who are alive five years after their diagnosis or the start of treatment. Hence, the "overall outcome" or "overall survival" is for a particular subject or patient the probability to survive over a certain time period, i.e. it is related to the life-expectancy of said subject or patient.

The "disease-specific survival" can be expressed as the "disease-specific survival rate" which is the percentage of people in a population (e.g. study or treatment group) who have not died from a specific disease in a defined period of time. The time period usually begins at the time of diagnosis or at the start of treatment and ends at the time of death. Patients who died from causes other than the disease being studied (here: non-neuroendocrine bladder cancer) are not counted in this measurement. Hence "disease-specific" survival or outcome means for an individual subject or patient the probability to not die from non-neuroendocrine bladder cancer in a defined period of time.
"Progression-free survival" is the length of time during and after the treatment of a disease, such as non-neuroendocrine bladder cancer, that a subject or patient lives with the disease but it does not get worse. "Progression" would, e.g., be any new appearance of tumor (at the primary or a distant site) ("recurrence") or an increase in size, histological stage, grade or symptom, particularly following a given curative or palliative treatment. Progression can for example be detected radiographically or biochemically. For instance, in the case of NMIBC, progression could refer to the progression from stage Ta to T1 to T2 to T3 to T4; or from stage G1/2 to G3, or from stage LN0 to LN+ or from stage M0 to M+. For non-metastatic MIBC, progression could refer to post-operative recurrence, progression from stage LN0 to LN+ or from stage M0 to M+. Disease-specific death can also be considered as progression. In metastatic MIBC, progression can for example be detected radiographically, e.g. according to the RECIST criteria.
In some aspects, the poor outcome is for example an increased risk for a reduced life expectancy, an increased risk of progression, an increased risk of cancer-related death and/or an increased risk of recurrence after surgical treatment and/or drug treatment and/or radiation therapy.

The methods and uses of the present invention can be applied in different situations and different stages of the disease, e.g. pre-interventional or post-interventional. The terms "pre-interventionally", "pre-interventional" and "before intervention" herein relate to the time before intervention for the treatment of non-neuroendocrine bladder cancer has been started. By "intervention" any medical intervention used to modify a health outcome is meant. This definition includes drug administration, surgical procedures, application of devices, behavioural treatments, process-of-care changes, and the like. Preferably, the sample is taken upon admission of the patient to a hospital or before the diagnosis of non-neuroendocrine bladder cancer has been confirmed.
For example, in one aspect the subject has been diagnosed with non-neuroendocrine bladder cancer and has (so far) not undergone surgical treatment of said non-neuroendocrine bladder cancer. In such a case, the methods of the invention may be used for the prediction of the outcome or for the selection of the appropriate treatment (e.g. surgical option).
The terms "post-interventionally", "post-interventional" and "after intervention" relate to the time after intervention or the treatment has been started.
For example, in one aspect the subject has already undergone surgical treatment of said non-neuroendocrine bladder cancer, e.g. radical cystectomy of said non-neuroendocrine bladder cancer. In such a case, the methods of the invention may be used for the prediction of the outcome and/or for the selection of an appropriate follow-up treatment and/or for monitoring the subject (e.g. for progression of the disease). Hence, the level of CgA (and any other additional marker including MMP7 as the case may be) may be used for post-operative control. The term "post-operative control" in the context of the present invention refers to the monitoring of said subject following a surgical procedure of said subject.

"Prediction" in the context of the present invention in one instance relates to prognosing a complication, progression or symptom before other symptoms or markers have become evident or have become significantly altered. In the context of the present invention, terms such as "predictive value" refer to the statistic significance of a certain determined result of a measurement. Thus, an increase in predictive value or predictive power in the context of the present invention means that the probability of a correct diagnosis, prognosis, stratification or the like based on a certain value determined from the measurement of the level of a certain marker in a sample increases.

As outlined herein above, the level of CgA in the sample of said subject (as well as the levels of any other additional markers such as MMP7) may be compared to a control level or a predetermined threshold. These control levels or predetermined thresholds may be absolute values or relative values.
Depending on the specific application, the control level may be the level of CgA in an earlier sample from the same subject, e.g. the control level may be the level of CgA in a sample taken from the same subject prior to surgical treatment (e.g. RCE), and the level determined may be from a sample taken after the surgical treatment (e.g. RCE). The same applies for other markers that may be additionally determined, e.g. MMP7.
The pre-determined threshold for the CgA level may for example be selected in the range of from 100 ng/mL to 431 ng/mL, preferably in the range of from 103 ng/mL to 191 ng/mL, more preferably 130 ng/mL to 160 ng/mL. and most preferably 147 ng/mL. The selection of the threshold for a specific application may for example be based on the desired specificity and/or selectivity of the assay; see below.

As outlined herein above, in addition to the level of CgA, the level of other markers, particularly biomarkers, may be determined from the same subject, preferably in the same sample. One such marker that has turned out to be of particular value together with CgA is matrix metalloproteinase-7 (MMP7). Hence, in the context of the present invention, preferably additionally the level of matrix metalloproteinase 7 (MMP7) is determined in said sample of said subject or another sample from said subject, and the level of MMP7 in the sample from the subject is indicative for the severity of the non-neuroendocrine bladder cancer and/or the outcome for the subject. An increased level of MMP7 in the sample from the subject as compared to a control level or a predetermined threshold may be indicative for a poor outcome for the subject, particularly an increased risk for a reduced life expectancy, an increased risk of progression, an increased risk of cancer-related death and/or an increased risk of recurrence after surgical treatment and/or drug treatment and/or radiation therapy. The level of MMP7 in the same sample or another sample from the subject may in one aspect be indicative for the subject's overall survival or the subject's disease-specific survival or the subject's progression-free survival. The pre-determined threshold for the MMP7 level may for example be selected in the range of from 4.4 to 21 ng/mL, preferably in the range of from 5.4 to 10.1 ng/mL, more preferably in the range of from 6 to 9 ng/mL. Most preferably, the predetermined threshold for the MMP7 level is 7.75 ng/mL. The selection of the threshold for a specific application may for example be based on the desired specificity and/or selectivity of the assay; see below.

In a particular embodiment of the methods of the present invention,
(i) a level of CgA in said sample above the predetermined CgA threshold is indicative of a high risk of cancer-related death after surgical treatment (e.g. TURBT, partial cystectomy (PCE), RCE, RCE combined with regional or extended LND, metastasectomy), or drug treatments (chemotherapy, targeted therapy, immunotherapy) or radiotherapy and
(ii) a level of CgA in said sample above the predetermined CgA threshold and a level of MMP7 in said sample above the predetermined MMP7 threshold is indicative of very high risk of cancer-related death and shorter lifetime after surgical treatment (e.g. TURBT, PCE, RCE, RCE combined with regional or extended LND, metastasectomy) or drug treatments (chemotherapy, targeted therapy, immunotherapy) or radiotherapy.

Using the methods of the present invention, the clinician is able to choose the most appropriate treatment based on the determined risk of the patient. As outlined, the invention provides a post-surgical, non-invasive, inexpensive and easy to handle tool to monitor treatment efficiency and the recurrence of bladder cancer, particularly non-neuroendocrine bladder cancer and preferably urothelial carcinoma. For example, the level of CgA and optionally MMP7 may be determined at regular time intervals during follow-up, and the control level may be the respective level determined before said surgical treatment and/or at the initial step of the follow-up.

In one particular aspect, the pre-operative level (preferably the serum level) of CgA is used as a marker for overall- and disease-specific survival in patients after surgery (e.g. RCE). In a particular aspect of the invention MMP7 is used as an additional marker for poor disease-specific survival in patients treated with RCE. MMP7 may also be used as an additional marker for muscle-invasive tumor and/or metastasis.

As discussed, in addition to the determination of the level of CgA, additionally the level of other markers, particularly biomarkers (including MMP7), and other clinical parameters of the subject may be determined. Such clinical parameter(s) of the subject may for example be selected from the group consisting of tumor histological subtype, metastasis status (lymph nodes and distant), lymphadenopathy, smoking or tobacco use, age, gender, family history, ethnicity, body weight, Body Mass Index (BMI), cystoscopy report, urine cytology (VUC), ultrasonography, CT scan, MRI and TURBT, and blood pressure. In a particular embodiment, additionally at least one clinical parameter is determined selected from the group comprising age, gender, systolic blood pressure, diastolic blood pressure, antihypertensive treatment, history of urinary tract diseases and treatments, history of stroke, wheezing, body mass index, heart rate, temperature, presence of diabetes mellitus and current smoking habits.

Additionally the level of a marker selected from the group consisting of Complement factor H-related protein and complement factor H, nuclear matrix protein BLCA-4, Survivin (BIRC5, EPR-1), Cytokeratin 8 (CK8), Cytokeratin 18 (CK18), Cytokeratin 20 (CK20), CEA protein and bladder tumor cell-associated mucins, alterations in chromosomes 3, 7, 17 and 9p21, CEA protein (CEA), CYFRA 21-1 (CK19), carbonic anhydrase, neurologic sensory protein (NSE), c-reactive protein (CRP), nuclear mitotic apparatus protein 22 (NMP22), alkalin phosphatase, matrix metalloproteinase 1 (MMP-1), matrix metalloproteinase 2 (MMP-2), matrix metalloproteinase 3 (MMP-3), matrix metalloproteinase 9 (MMP-9), matrix metalloproteinase 10 (MMP-10), matrix metalloproteinase 13 (MMP-13), matrix metalloproteinase 26 (MMP-26), tissue inhibitor of metalloproteinases 1 (TIMP-1), tissue inhibitor of metalloproteinases 2 (TIMP-2), tissue inhibitor of metalloproteinases 3 (TIMP-3), tissue inhibitor of metalloproteinases 4 (TIMP-4), alpha-1 antitrypsin, vascular endothelial growth factor (VEGF), placental growth factor (PLGF), vascular endothelial growth factor receptor 1 (VEGFR-1), soluble vascular endothelial growth factor receptor 1 (sVEGFR-1, sfLT-1), vascular endothelial growth factor receptor 2 (VEGFR-2), soluble vascular endothelial growth factor receptor 3 (sVEGFR-3), vascular endothelial growth factor A (VEGFA), vascular endothelial growth factor C (VEGFC), insulin-like growth factor binding protein 1 (IGFBP-1), insulin-like growth factor binding protein 2 (IGFBP-2), insulin-like growth factor binding protein 3 (IGFBP-3), insulin-like growth factor binding protein 4 (IGFBP-4), insulin-like growth factor binding protein 5 (IGFBP-5), insulin-like growth factor binding protein 6 (IGFBP-6), transforming growth factor β (TGF-β), insulin-like growth factor (IGF), insulin-like growth factor receptor (IGFR), insulin-like growth factor 1 (IGF-1), insulin-like growth factor 1 receptor (IGF1R), endothelial growth factor (EGF), endothelial growth factor receptor (EGFR), proheparin-binding EGF- like growth factor (proHB-EGF), insulin-like growth factor 2 mRNA-binding protein 3 (IGFBP3), insulin-like growth factor 2 mRNA-binding protein 7 (IGFBP7), angiostatin, endostatin, plasminogen (PLG), aquaporin 1 (AQP-1), perilipin 2 (PLIN-2), human chorionic gonadotropin (hCG), androgen receptor (AR), estrogen receptor (ER), prostate-specific antigen (PSA), free prostate-specific antigen (free PSA), total prostate-specific antigen (total PSA), tumor necrosis factor α (TNFα), E-cadherin, elastin, fibronectin, collagen, and vitronectin may be determined in said sample or another sample of the subject. Among these, the preferred additional markers are selected from Complement factor H-related protein and complement factor H, nuclear matrix protein BLCA-4, Cytokeratin 8 (CK8), Cytokeratin 18 (CK18), CEA protein and bladder tumor cell-associated mucins, alterations in chromosomes 3, 7, 17 and 9p21, CEA protein (CEA), nuclear mitotic apparatus protein 22 (NMP22), alkalin phosphatase, tissue inhibitor of metalloproteinases 1 (TIMP-1), and tissue inhibitor of metalloproteinases 2 (TIMP-2).

The term "biomarker" (biological marker) relates to measurable and quantifiable biological parameters (e.g., specific enzyme concentration, specific hormone concentration, specific gene phenotype distribution in a population, presence of biological substances) which serve as indices for health- and physiology-related assessments, such as disease risk, psychiatric disorders, environmental exposure and its effects, disease diagnosis, metabolic processes, substance abuse, pregnancy, cell line development, epidemiologic studies, etc. Furthermore, a biomarker is defined as a characteristic that is objectively measured and evaluated as an indicator of normal biological processes, pathogenic processes, or pharmacologic responses to a therapeutic intervention. A biomarker may be measured on a biosample (e.g. whole blood, serum, plasma urine), it may be a recording obtained from a person (blood pressure, ECG, or Holter), or it may be an imaging test (echocardiogram or CT scan) (Vasan et al. 2006, Circulation 113:2335-2362). Biomarkers can indicate a variety of health or disease characteristics, including the level or type of exposure to an environmental factor, genetic susceptibility, genetic responses to exposures, biomarkers of subclinical or clinical disease, or indicators of response to therapy. Thus, a simplistic way to think of biomarkers is as indicators of disease trait (risk factor or risk biomarker), disease state (preclinical or clinical), or disease rate (progression). Accordingly, biomarkers can be classified as antecedent biomarkers (identifying the risk of developing an illness), screening biomarkers (screening for subclinical disease), diagnostic biomarkers (recognizing overt disease), staging biomarkers (categorizing disease severity), or prognostic biomarkers (predicting future disease course, including recurrence and response to therapy, and monitoring efficacy of therapy). Biomarkers may also serve as surrogate end points. A surrogate end point is one that can be used as an outcome in clinical trials to evaluate safety and effectiveness of therapies in lieu of measurement of the true outcome of interest. The underlying principle is that alterations in the surrogate end point track closely with changes in the outcome of interest. Surrogate end points have the advantage that they may be gathered in a shorter time frame and with less expense than end points such as morbidity and mortality, which require large clinical trials for evaluation. Additional values of surrogate end points include the fact that they are closer to the exposure/intervention of interest and may be easier to relate causally than more distant clinical events. An important disadvantage of surrogate end points is that if clinical outcome of interest is influenced by numerous factors (in addition to the surrogate end point), residual confounding may reduce the validity of the surrogate end point. It has been suggested that the validity of a surrogate end point is greater if it can explain at least 50% of the effect of an exposure or intervention on the outcome of interest. For instance, a biomarker may be a protein, peptide or a nucleic acid molecule. The National Institute of Health (NIH) defines a biomarker as a biological marker that is objectively measured and evaluated as an indicator of a normal biological process, pathogenic process, or pharmacological responses to therapeutic interventions (Danesh et al. Clin Pharmacol Ther 2001. 169:416-468).
Some of the markers (particularly biomarkers) are "cancer markers", i.e. they are markers associated with the diagnosis and/or prognosis of cancer, here: non-neuroendocrine bladder cancer.

The methods of the present invention may comprise the initial step that a sample of a bodily fluid of said subject is to be provided. The term "sample" as used in the context of the present invention refers to a sample of bodily fluid obtained for the purpose of diagnosis, prognosis, or evaluation of a subject of interest, such as a patient. Preferred test samples include (whole) blood, serum, plasma and urine. Some test samples are more readily analyzed following a fractionation or purification procedure, for example, separation of whole blood into serum or plasma components. Thus, in a preferred embodiment of the invention the sample is selected from the group comprising a (whole) blood sample, a serum sample, a plasma sample and a urine sample or an extract of any of the aforementioned samples. Preferably, the sample is a whole blood sample, most preferably a serum sample or a plasma sample. As used herein, "whole blood sample" specifies an unprocessed or essentially unprocessed blood sample. Where appropriate, the sample may need to be homogenized, or extracted with a solvent prior to use in the present invention in order to obtain a liquid sample. A liquid sample hereby may be a solution or suspension. Liquid samples may be subjected to one or more pre-treatments prior to use in the present invention. Such pre-treatments include, but are not limited to dilution, filtration, centrifugation, concentration, sedimentation, precipitation, or dialysis. Pre-treatments may also include the addition of chemical or biochemical substances to the solution, such as acids, bases, buffers, salts, solvents, reactive dyes, detergents, emulsifiers or chelators.
"Plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood) for at least 15 minutes at 2000 to 3000 g. Therefore, it is preferred that plasma samples employed in the context of the present invention have been subjected to centrifugation at more than 1500g for 30 min, preferably at least at 2000g for at least 30 min, more preferably at least at 3000g for at least 20 min, most preferably at least at 3000g for at least 30 min.
"Serum" in the context of the present invention is the undiluted, extracellular portion of blood after adequate coagulation is completed. Coagulation is usually completed after 30 min. Serum can be obtained by centrifugation of the coagulated sample for at least 10 minutes at a minimum speed of 1500 g. Therefore, it is preferred that serum samples employed in the context of the present invention have been subjected to centrifugation at least at 1500g for at least 10 min, preferably for at least 15 min, more preferably for at least 20 min. Most preferably the serum sample has been subjected to a centrifugation at least at 3000g for at least 20 min.

The level of CgA can be determined using appropriate assays. An "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably greater than 10⁸ M⁻¹.

The preferred detection methods comprise immunoassays in various formats such as for instance radioimmunoassay (RIA), chemiluminescence- and fluorescence-immunoassays, Enzyme-linked immunoassays (ELISA), Luminex-based bead arrays, protein microarray assays, rapid test or point-of-care (PoC) formats such as for instance immunochromatographic strip tests and automated immunoassay systems such as for instance the BRAHMS KRYPTOR System. Especially preferred are PoC test formats; e.g. see St. John & Price, Clin Biochem Rev. 2014 Aug; 35(3): 155-167. Moreover, mass spectrometry approaches can be used to detect and quantify CgA, MMP7 and/or further biomarkers, e.g. quantitative selected reaction monitoring (qSRM) can be used. For mass spectrometric measurements, chemical derivatization is usually performed before analyzing and quantifying the target protein.

The immunoassays can be homogenous or heterogeneous assays, competitive and non-competitive assays. In a particularly preferred embodiment, the assay is in the form of a sandwich assay, which is a non-competitive immunoassay, wherein the molecule to be detected and/or quantified is bound to a first antibody and to a second antibody. The first antibody may be bound to a solid phase, e.g. a bead, a surface of a well or other container, a chip or a strip, and the second antibody is an antibody which is labelled, e.g. with a dye, with a radioisotope, or a reactive or catalytically active moiety. The amount of labelled antibody bound to the analyte is then measured by an appropriate method. The general composition and procedures involved with "sandwich assays" are well-established and known to the skilled person (The Immunoassay Handbook, Ed. David Wild, Elsevier LTD, Oxford; 3rd ed. (May 2005), ISBN-13: 978-0080445267; Hultschig C et al., Curr Opin Chem Biol. 2006 Feb;10(1):4-10).

In a particularly preferred embodiment the assay comprises two capture molecules, preferably antibodies which are both present as dispersions in a liquid reaction mixture, wherein a first labelling component is attached to the first capture molecule, wherein said first labelling component is part of a labelling system based on fluorescence- or chemiluminescence-quenching or amplification, and a second labelling component of said marking system is attached to the second capture molecule, so that upon binding of both capture molecules to the analyte a measurable signal is generated that allows for the detection of the formed sandwich complexes in the solution comprising the sample.
Even more preferred, said labelling system comprises rare earth cryptates or rare earth chelates in combination with fluorescence dye or chemiluminescence dye, in particular a dye of the cyanine type.

In the context of the methods of the present invention, the level of CgA and/or MMP7 is preferably determined using an immunoassay. Suitable immunoassays and antibodies for the detection of CgA are for example described in WO 2015/158701 A1 and Popovici et al. (2014. Clin Biochem 47: 87-91). For example, the B·R·A·H·M·S Chromogranin A KRYPTOR assay (B·R·A·H·M·S GmbH, Hennigsdorf, Germany) can be used. Further commercial assays for the detection of Chromogranin A are available and can be used: The Cis-Bio ELISA assay (Cisbio Bioassays, Codolet, France) uses two monoclonal antibodies directed against epitopes corresponding to amino acids 145-197 and 219-234, the DAKO ELISA assay (Dako Denmark A/S, Glostrup, Denmark) uses rabbit polyclonal antibodies directed against a 23 kDa C-terminal fragment, the Euro-Diagnostica NEOLISA™ sandwich ELISA assay (Euro Diagnostica AB, Malmö, Sweden) uses two monoclonal antibodies directed against epitopes corresponding to amino acids 236-251 and 264-279 (also see WO 2011/135035 A1 and WO 99/58980 A1).
Suitable immunoassays for the detection of MMP7 are for example described in WO 2007/144144 A1. Commercially available assays for MMP7 include: Human Total MMP-7 Quantikine ELISA Kit or Human Magnetic Luminex Screening Assay or Human Luminex Screening Assay or Human MMP-7 Luminex Performance Assay or Human MMP-7 Magnetic Luminex Performance Assay or Human Total MMP-7 DuoSet ELISA or Human MMP Premixed Luminex Performance Assay Kit or Proteome Profiler Human Protease Array Kit (R&D Systems Minneapolis, MN 55413, USA), Human Total MMP-7 ELISA Kit (Aviva Systems Biology Corporation , San Diego, CA 92121, USA), MMP7 (Human) ELISA Kit (Abnova, Taipei 114, Taiwan), Human MMP7 / Matrilysin ELISA Kit (Sandwich ELISA) (LifeSpan Biosciences, Seattle, Washington 98121, USA), MMP7 Human ELISA Kit (Abcam, Cambridge , United Kingdom), SensoLyte® 490 MMP - 7 Assay Kit *Fluorimetric* (ANASPEC, Fremont, CA 94555, USA), Bio-Plex Pro™ Human MMP and TIMP Assays (Bio-Rad Laboratories, Inc., Hercules, CA, U.S.A.), ELISA Kit for Matrix Metalloproteinase 7 (MMP7) (Wuhan USCN Business Co., Ltd., Wuhan City, China).

The immunoassays for the detection of CgA described in WO 2015/158701 A1 are preferred in the context of the present invention. They are based on the use of two antibodies against CgA (sandwich immunoassay). The (first and/or second) antibodies or antigen-binding fragments or derivatives thereof of the immunoassay method as described in WO 2015/158701 A1 may for instance be polyclonal antibodies, monoclonal antibodies or genetically engineered monoclonal antibodies. Said first antibody is specific for an epitope in the sequence of CgA (SEQ ID NO:1), preferably in the sequence spanning amino acids 124 to 144 of SEQ ID NO:1. The first antibody is preferably a monoclonal antibody. Said second antibody is specific for an epitope in the sequence of CgA (SEQ ID NO:1), preferably in the sequence spanning amino acids 280 to 301 of SEQ ID NO:1. The second antibody is preferably a monoclonal antibody. In a particular immunoassay, the first antibody is specific for an epitope in the sequence of CgA (SEQ ID NO:1) spanning amino acid residues 124-144 and the second antibody is specific for an epitope in the sequence of CgA (SEQ ID NO:1) spanning amino acid residues 280 to 301. The first and second antibodies are preferably monoclonal antibodies. The first antibody or antigen-binding fragment or derivative thereof may for example be produced by the hybridoma cell line 537/H2 deposited as DSM ACC3231. The antibody produced by hybridoma cell line 537/H2 binds specifically to amino acid residues 124 to 144 of the CgA sequence (SEQ ID NO:1). The second antibody or antigen-binding fragment or derivative thereof may for example be produced by the hybridoma cell line 541/E2 deposited as DSM ACC3232. The antibody produced by hybridoma cell line 541/E2 binds specifically to amino acid residues 280 to 301 of the CgA sequence (SEQ ID NO:1). In a particular embodiment of the immunoassay, the first antibody is produced by the hybridoma cell line 537/H2 deposited as DSM ACC3231 and the second antibody is produced by the hybridoma cell line 541/E2 deposited as DSM ACC3232.

Biomarkers such as CgA may be fragmented into shorter peptides or proteins. Hence, in some instances also fragments of CgA or other peptide biomarkers such as MMP7 may be detected and their level in the sample may be determined. The term "fragment" refers to smaller proteins or peptides derivable from larger proteins or peptides, which hence comprise a partial sequence of the larger protein or peptide. Said fragments are derivable from the larger proteins or peptides by saponification of one or more of its peptide bonds. Said peptide fragments have preferably a length of at least about 15 to about 20 amino acids, more preferably at least about 25 to about 45 amino acids.

The measured levels of the markers (CgA, MMP7 and others as the case may be) can be correlated to a certain diagnosis and/or prognosis, e.g. using a particular mathematical algorithm. In the context of the present invention, an "algorithm" or "mathematical algorithm" refers to the use of a mathematical or statistical method or model used to compare a certain measured value with values of a reference population in order to stratify said measured value. This may for instance be the median of the level of a certain entity in an ensemble of pre-determined samples, which means that the measured level of said entity is compared with the mathematical median of the level of said entity in a given number of samples. The number of samples used to determine the median is not particularly limited, but should be sufficient in order to ensure statistical significance of the median. The number of samples used to determine the median may even increase over the course of time, as the results of further measurement values from clinical samples are added in order to increase the statistic significance of the median. Preferably, the sample number is chosen such that statistical significance of the median is ensured. Thus, said median is used as a reference value, whereby the measured level of the aforementioned entity can be statistically correlated with a certain physiological state, e.g. the propensity of an adverse outcome for a patient, depending on the relative level above or below the median and the extent of deviation of the measured value from said median. In place of the median, other statistical methods, such as the determination of quantiles (e.g. quartiles or percentiles) or mathematical models, preferably Cox Regression may be used analogously to the above description in order to obtain the above-mentioned reference value and/or otherwise determine the significance of a measured value with respect to the physiological status of a given subject from which the sample has been obtained. Said mathematical or statistical methods or models are well known to the person skilled in the art and the use thereof in the context of medicinal applications is well established.

The term "correlated" or "correlating", as used herein in reference to the use of diagnostic and prognostic marker(s), refers to comparing the presence or level of the marker(s) in a patient to its presence or amount in persons known to suffer from, or known to be at risk of, a given condition. A marker level in a patient sample can be compared to a level known to be associated with a specific diagnosis. The sample's marker level is said to have been correlated with a diagnosis; that is, the skilled artisan can use the marker level to determine whether the patient suffers from a specific type of a disease, and respond accordingly. Alternatively, the sample's marker level can be compared to a marker level known to be associated with a good outcome (e.g. the absence of disease etc.). In preferred embodiments, a panel of marker levels is correlated to a global probability or a particular outcome.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group. In certain preferred embodiments, markers and/or marker panels are preferably selected to exhibit a positive or negative likelihood ratio of at least about 1.5 or more or about 0.67 or less, more preferably at least about 2 or more or about 0.5 or less, still more preferably at least about 5 or more or about 0.2 or less, even more preferably at least about 10 or more or about 0.1 or less, and most preferably at least about 20 or more or about 0.05 or less. The term "about" in this context refers to +/- 5% of a given measurement.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In certain preferred embodiments, markers and/or marker panels are preferably selected to exhibit an odds ratio of at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less. The term "about" in this context refers to +/- 5% of a given measurement.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group. In certain preferred embodiments, markers and/or marker panels are preferably selected to exhibit a hazard ratio of at least about 1.1 or more or about 0.91 or less, more preferably at least about 1.25 or more or about 0.8 or less, still more preferably at least about 1.5 or more or about 0.67 or less, even more preferably at least about 2 or more or about 0.5 or less, and most preferably at least about 2.5 or more or about 0.4 or less. The term "about" in this context refers to +/-5% of a given measurement.

The sensitivity and specificity of a diagnostic and/or prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy) and "disease" populations (i.e. patients suffering from bladder cancer, particularly non-neuroendocrine bladder cancer and preferably urothelial carcinoma). For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results don't necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art (See, e.g., Hanley et al.1982. Radiology 143: 29-36). Preferably, ROC curves result in an AUC of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In certain embodiments, markers and/or marker panels are selected to exhibit at least about 70% sensitivity, more preferably at least about 80% sensitivity, even more preferably at least about 85% sensitivity, still more preferably at least about 90% sensitivity, and most preferably at least about 95% sensitivity, combined with at least about 70% specificity, more preferably at least about 80% specificity, even more preferably at least about 85% specificity, still more preferably at least about 90% specificity, and most preferably at least about 95% specificity. In particularly preferred embodiments, both the sensitivity and specificity are at least about 75%, more preferably at least about 80%, even more preferably at least about 85%, still more preferably at least about 90%, and most preferably at least about 95%. The term "about" in this context refers to +/- 5% of a given measurement.

Suitable threshold levels for the "stratification of subjects" into different groups (categories) have to be determined for each particular combination of CgA level, further markers (such as MMP7) and/or parameters, medication and disease. This can e.g. be done by grouping a reference population of patients according to their level of CgA into certain quantiles, e.g. quartiles, quintiles or even according to suitable percentiles. For each of the quantiles or groups above and below certain percentiles, hazard ratios can be calculated comparing the risk for an adverse outcome, i.e. an "unfavourable effect", e.g. in terms of survival rate, between those patients who have received a certain medication and those who did not. In such a scenario, a hazard ratio (HR) above 1 indicates a higher risk for an adverse outcome for the patients who have received a treatment than for patients who did not. A HR below 1 indicates beneficial effects of a certain treatment in the group of patients. A HR around 1 (e.g. +/- 0.1) indicates no elevated risk but also no benefit from medication for the particular group of patients. By comparison of the HR between certain quantiles of patients with each other and with the HR of the overall population of patients, it is possible to identify those quantiles of patients who have an elevated risk and those who benefit from medication and thereby stratify subjects according to the present invention.

The term "stratification to a therapeutic treatment" or "therapy stratification" in the context of the present invention refers to the assessment of an appropriate therapeutic treatment of said patient. Sub-classification relates to further defining a diagnosis according to different subclasses of the diagnosed disease, disorder, complication or risk, e.g. defining according to mild and severe forms of the disease. The term "therapy monitoring" in the context of the present invention refers to the control and/or adjustment of a therapeutic treatment of said patient.

The present disclosure also relates to methods for treating non-neuroendocrine bladder cancer, wherein the level of CgA (and optionally further markers and/or clinical parameters such as the level of MMP7) are determined in order to determine the most appropriate treatment. For example, a patient which has according to the determined increased level of CgA a relatively poor prognosis can benefit from a more aggressive treatment, e.g. partial cystectomy instead of TURBT or trimodality bladder preservation, RCE instead of TURBT or trimodality bladder preservation or partial cystectomy, RCE combined with PLND instead of RCE, extended or super extended LND instead of PLND, adjuvant chemotherapy or radiotherapy instead of surgery alone, additional metastasectomy. This high-risk patient could also be selected for clinical studies testing novel drugs.

Further, the present invention relates to the use of kits comprising antibodies specific for CgA and optionally antibodies specific for MMP7 for the the in vitro prognosis, risk assessment, risk stratification, monitoring and/or therapy control of non-neuroendocrine bladder cancer and preferably urothelial carcinoma. The kits may also comprise additional components such as buffers and instructions for use. A preferred kit for the detection of CgA is described in WO 2015/158701 A1 and comprises
(i) a first antibody or antigen-binding fragment or derivative thereof which is specific for CgA or a fragment thereof; and
(ii) a second antibody or antigen-binding fragment or derivative thereof which is specific for CgA or a fragment thereof.
The first and second antibodies of the kit are preferably specific for the same CgA fragment or fragments. For example, (i) the first antibody or antigen-binding fragment or derivative thereof is specific for an epitope comprised within the sequence of SEQ ID NO:1; and/or (ii) the second antibody or antigen-binding fragment or derivative thereof is specific for an epitope comprised within the sequence of SEQ ID NO:1. Preferably, the first antibody of the kit is a monoclonal anti- CgA antibody produced by hybridoma cell line 537/H2 deposited as DSM ACC3231 and/or the second antibody is a monoclonal anti- CgA antibody produced by hybridoma cell line 541/E2 deposited as DSM ACC3232.
The kit may comprise one or more antibodies for the detection of MMP7. For example, the kit (additionally) comprises
(i) a first antibody or antigen-binding fragment or derivative thereof which is specific for MMP7 or a fragment thereof; and
(ii) a second antibody or antigen-binding fragment or derivative thereof which is specific for MMP7 or a fragment thereof.
The first and second antibodies of the kit are preferably specific for the same MMP7 fragment or fragments. For example, (i) the first antibody or antigen-binding fragment or derivative thereof is specific for an epitope comprised within the sequence of SEQ ID NO:2; and/or (ii) the second antibody or antigen-binding fragment or derivative thereof is specific for an epitope comprised within the sequence of SEQ ID NO:2.

### Cited References

All references cited herein are hereby listed:
Alijo Serrano, F., N. Sanchez-Mora, J. Angel Arranz, C. Hernandez & E. Alvarez-Fernandez (2007) Large cell and small cell neuroendocrine bladder carcinoma: immunohistochemical and outcome study in a single institution. Am J Clin Pathol, 128, 733-9.
Babjuk, M. (2009) Transurethral Resection of Non-muscle-invasive Bladder Cancer. European Urology Supplements, 8, 542-548.
Bagchi, A., K. Dushaj, A. Shrestha, A. L. Leytin, S. A. Bhuiyan, F. Radparvar, S. Topchik, S. S. Tuli, P. Kim & S. Bakshi (2015) Urinary bladder paraganglioma presenting as micturition-induced palpitations, dyspnea, and angina. Am J Case Rep, 16, 283-6.
Bertaccini, A., D. Marchiori, A. Cricca, M. Garofalo, C. Giovannini, F. Manferrari, T. G. Gerace, R. Pernetti & G. Martorana (2008) Neuroendocrine carcinoma of the urinary bladder: case report and review of the literature. Anticancer Res, 28, 1369-72.
Brausi, M., J. A. Witjes, D. Lamm, R. Persad, J. Palou, M. Colombel, R. Buckley, M. Soloway, H. Akaza & A. Bohle (2011) A review of current guidelines and best practice recommendations for the management of nonmuscle invasive bladder cancer by the International Bladder Cancer Group. JUrol, 186, 2158-67.
Brausi, M. A. (2013) Primary prevention and early detection of bladder cancer: two main goals for urologists. Eur Urol, 63, 242-3.
Burger, M., J. W. Catto, G. Dalbagni, H. B. Grossman, H. Herr, P. Karakiewicz, W. Kassouf, L. A. Kiemeney, C. La Vecchia, S. Shariat & Y. Lotan (2013a) Epidemiology and risk factors of urothelial bladder cancer. Eur Urol, 63, 234-41.
Burger, M., W. Oosterlinck, B. Konety, S. Chang, S. Gudjonsson, R. Pruthi, M. Soloway, E. Solsona, P. Sved, M. Babjuk, M. A. Brausi, C. Cheng, E. Comperat, C. Dinney, W. Otto, J. Shah, J. Thurof, J. A. Witjes & C. International Consultation on Urologic Disease-European Association of Urology Consultation on Bladder (2013b) ICUD-EAU International Consultation on Bladder Cancer 2012: Non-muscle-invasive urothelial carcinoma of the bladder. Eur Urol, 63, 36-44.
Cauberg Evelyne, C. C., J. J. de la Rosette & T. M. de Reijke (2011) Emerging optical techniques in advanced cystoscopy for bladder cancer diagnosis: A review of the current literature. Indian J Urol, 27, 245-51.
Cerulli, C., G. M. Busetto, G. Antonini, R. Giovannone, M. Di Placido, G. Soda, E. De Berardinis & V. Gentile (2012) Primary metastatic neuroendocrine small cell bladder cancer: a case report and literature review. Urol Int, 88, 365-9.
Chamie, K., C. S. Saigal, J. Lai, J. M. Hanley, C. M. Setodji, B. R. Konety, M. S. Litwin & P. Urologic Diseases in America (2011) Compliance with guidelines for patients with bladder cancer: variation in the delivery of care. Cancer, 117, 5392-401.
Chuang, C. K. & S. K. Liao (2003) A retrospective immunohistochemical and clinicopathological study of small cell carcinomas of the urinary tract. Chang Gung Med J, 26, 26-33.
Clark, P. E., N. Agarwal, M. C. Biagioli, M. A. Eisenberger, R. E. Greenberg, H. W. Herr, B. A. Inman, D. A. Kuban, T. M. Kuzel, S. M. Lele, J. Michalski, L. C. Pagliaro, S. K. Pal, A. Patterson, E. R. Plimack, K. S. Pohar, M. P. Porter, J. P. Richie, W. J. Sexton, W. U. Shipley, E. J. Small, P. E. Spiess, D. L. Trump, G. Wile, T. G. Wilson, M. Dwyer & M. Ho (2013) Bladder cancer. J Natl Compr Canc Netw, 11, 446-75.
E1 Demery, M., G. Demirdjian-Sarkissian, S. Thezenas, W. Jacot, Y. Laghzali, B. Darbouret, S. Culine, X. Rebillard & P.-J. Lamy (2014) Serum Matrix Metalloproteinase-7 is an independent prognostic biomarker in advanced bladder cancer. Clinical and Translational Medicine, 3*.*
Feng, N., X. Li, H. D. Gao, Z. L. Liu, L. J. Shi & W. Z. Liu (2013) Urinary bladder malignant paraganglioma with vertebral metastasis: a case report with literature review. Chin J Cancer, 32, 624-8.
Fernandez-Gomez, J., R. Madero, E. Solsona, M. Unda, L. Martinez-Pineiro, A. Ojea, J. Portillo, M. Montesinos, M. Gonzalez, C. Pertusa, J. Rodriguez-Molina, J. E. Camacho, M. Rabadan, A. Astobieta, S. Isorna, P. Muntanola, A. Gimeno, M. Blas, J. A. Martinez-Pineiro & O. Club Urologico Espanol de Tratamiento (2011) The EORTC tables overestimate the risk of recurrence and progression in patients with non-muscle-invasive bladder cancer treated with bacillus Calmette-Guerin: external validation of the EORTC risk tables. Eur Urol, 60, 423-30.
Goodison, S., C. J. Rosser & V. Urquidi (2013) Bladder cancer detection and monitoring: assessment of urine- and blood-based marker tests. Mol Diagn Ther, 17, 71-84.
Gunes, M., A. S. Kemik, N. Pirincci, I. Gecit, K. Taken, M. B. Yuksel, M. Kaba & R. Eryilmaz (2013) Preoperative levels of matrix metalloproteinase-7 and -9 and tissue inhibitor of matrix metalloproteinase-1 relation to pathologic parameters in bladder carcinoma patients. Asian Pac J Cancer Prev, 14, 873-6.
Hansel, D. E., J. S. Miller, M. S. Cookson & S. S. Chang (2013) Challenges in the Pathology of Non-Muscle-invasive Bladder Cancer: A Dialogue Between the Urologic Surgeon and the Pathologist. Urology*.*
Iczkowski, K. A., J. H. Shanks, W. C. Allsbrook, A. Lopez-Beltran, C. G. Pantazis, T. R. Collins, R. W. Wetherington & D. G. Bostwick (1999) Small cell carcinoma of urinary bladder is differentiated from urothelial carcinoma by chromogranin expression, absence of CD44 variant 6 expression, a unique pattern of cytokeratin expression, and more intense gamma-enolase expression. Histopathology, 35, 150-6.
Jager, T., S. Tschirdewahn, F. Vom Dorp, G. Piechotta, H. Rubben & T. Szarvas (2013) [Siliconchiptechnology-based MMP-7 analysis in urine : An option for preoperative identification of lymph node metastasis in bladder cancer]. Urologe A, 52, 853-8.
Kamat, A. M., P. K. Hegarty, J. R. Gee, P. E. Clark, R. S. Svatek, N. Hegarty, S. F. Shariat, E. Xylinas, B. J. Schmitz-Drager, Y. Lotan, L. C. Jenkins, M. Droller, B. W. van Rhijn, P. I. Karakiewicz & C. International Consultation on Urologic Disease-European Association of Urology Consultation on Bladder (2013) ICUD-EAU International Consultation on Bladder Cancer 2012: Screening, diagnosis, and molecular markers. Eur Urol, 63, 4-15.
Leal, J., R. Luengo-Fernandez, R. Sullivan & J. A. Witjes (2015) Economic Burden of Bladder Cancer Across the European Union. Eur Urol*.*
Maurer, T., T. Horn, M. Heck, J. Gschwend, M. Eiber & A. Beer (2013) Current Staging Procedures in Urinary Bladder Cancer. Diagnostics, 3, 315-324.
Mossanen, M. & J. L. Gore (2014) The burden of bladder cancer care: direct and indirect costs. Curr Opin Urol*.*
Pompas-Veganzones, N., P. Gonzalez-Peramato & M. Sanchez-Carbayo (2014) The neuroendocrine component in bladder tumors. Curr Med Chem, 21, 1117-28.
Scarpato, K. R., A. K. Morgans & K. A. Moses (2015) Optimal management of muscle-invasive bladder cancer - a review. Res Rep Urol, 7, 143-51.
Schmitz-Drager, B. J., M. Droller, V. B. Lokeshwar, Y. Lotan, M. A. Hudson, B. W. van Rhijn, M. J. Marberger, Y. Fradet, G. P. Hemstreet, P. U. Malmstrom, O. Ogawa, P. I. Karakiewicz & S. F. Shariat (2015) Molecular markers for bladder cancer screening, early diagnosis, and surveillance: the WHO/ICUD consensus. Urol Int, 94, 1-24.
Shariat, S. F., B. Ehdaie, M. Rink, E. K. Cha, R. S. Svatek, T. F. Chromecki, H. Fajkovic, G. Novara, S. G. David, S. Daneshmand, Y. Fradet, Y. Lotan, A. I. Sagalowsky, T. Clozel, P. J. Bastian, W. Kassouf, H. M. Fritsche, M. Burger, J. I. Izawa, D. Tilki, F. Abdollah, F. K. Chun, G. Sonpavde, P. I. Karakiewicz, D. S. Scherr & M. Gonen (2012) Clinical nodal staging scores for bladder cancer: a proposal for preoperative risk assessment. Eur Urol, 61, 237-42.
Sharma, S., P. Ksheersagar & P. Sharma (2009) Diagnosis and treatment of bladder cancer. Am Fam Physician, 80, 717-23.
Skinner, E. C. & A. I. Sagalowsky (2014) Is extended lymphadenectomy of beneficial therapeutic value for T2 urothelial cancer? J Urol, 191, 1206-8.
Soukup, V., M. Kalousova, O. Capoun, R. Sobotka, Z. Breyl, M. Pesl, T. Zima & T. Hanus (2015) Panel of Urinary Diagnostic Markers for Non-Invasive Detection of Primary and Recurrent Urothelial Urinary Bladder Carcinoma. Urol Int, 95, 56-64.
Svatek, R. S., B. K. Hollenbeck, S. Holmang, R. Lee, S. P. Kim, A. Stenzl & Y. Lotan (2014) The Economics of Bladder Cancer: Costs and Considerations of Caring for This Disease. Eur Urol*.*
Svatek, R. S., J. B. Shah, J. Xing, D. Chang, J. Lin, D. J. McConkey, X. Wu & C. P. Dinney (2010) A multiplexed, particle-based flow cytometric assay identified plasma matrix metalloproteinase-7 to be associated with cancer-related death among patients with bladder cancer. Cancer, 116, 4513-9.
Szarvas, T., M. Becker, F. vom Dorp, C. Gethmann, M. Totsch, A. Bankfalvi, K. W. Schmid, I. Romics, H. Rubben & S. Ergun (2010) Matrix metalloproteinase-7 as a marker of metastasis and predictor of poor survival in bladder cancer. Cancer Sci, 101, 1300-8.
Szarvas, T., T. Jager, M. Becker, S. Tschirdewahn, C. Niedworok, I. Kovalszky, H. Rubben, S. Ergun & F. vom Dorp (2011a) Validation of circulating MMP-7 level as an independent prognostic marker of poor survival in urinary bladder cancer. Pathol Oncol Res, 17, 325-32.
Szarvas, T., B. B. Singer, M. Becker, F. Vom Dorp, T. Jager, A. Szendroi, P. Riesz, I. Romics, H. Rubben & S. Ergun (2011b) Urinary matrix metalloproteinase-7 level is associated with the presence of metastasis in bladder cancer. BJU Int, 107, 1069-73.
Vedder, M. M., M. Marquez, E. W. de Bekker-Grob, M. L. Calle, L. Dyrskjot, M. Kogevinas, U. Segersten, P. U. Malmstrom, F. Algaba, W. Beukers, T. F. Orntoft, E. Zwarthoff, F. X. Real, N. Malats & E. W. Steyerberg (2014) Risk prediction scores for recurrence and progression of non-muscle invasive bladder cancer: an international validation in primary tumours. PLoS One, 9, e96849.
Witjes, J. A., E. Comperat, N. C. Cowan, M. De Santis, G. Gakis, T. Lebret, M. J. Ribal, A. G. Van der Heijden & A. Sherif (2014) EAU guidelines on muscle-invasive and metastatic bladder cancer: summary of the 2013 guidelines. Eur Urol, 65, 778-92.
Xylinas, E., M. Kent, L. Kluth, A. Pycha, E. Comploj, R. S. Svatek, Y. Lotan, Q. D. Trinh, P. I. Karakiewicz, S. Holmang, D. S. Scherr, M. Zerbib, A. J. Vickers & S. F. Shariat (2013) Accuracy of the EORTC risk tables and of the CUETO scoring model to predict outcomes in non-muscle-invasive urothelial carcinoma of the bladder. Br J Cancer, 109, 1460-6.
Yafi, F. A., A. G. Aprikian, J. L. Chin, Y. Fradet, J. Izawa, E. Estey, A. Fairey, R. Rendon, I. Cagiannos, L. Lacombe, J. B. Lattouf, D. Bell, D. Drachenberg & W. Kassouf(2011) Contemporary outcomes of 2287 patients with bladder cancer who were treated with radical cystectomy: a Canadian multicentre experience. BJU Int, 108, 539-45.

### Sequences

### Examples

### Example 1: Detection of Chromogranin A and MMP7 in bladder cancer patients Study design

Serum samples were collected in 188 patients who underwent surgical treatment of urinary bladder cancer (BCa): transurethral resection of the bladder (TURB) or radical cystectomy (RCE). Patients were enrolled in the Department of Urology of the University Hospital Duisburg-Essen between August 2008 and November 2011. The criteria for study enrolment were histopathological diagnosis of transitional cell carcinoma of the bladder, no history of other tumor, no chemotherapy before surgery, availability of sufficient serum sample and the potential to follow up. No neuroendocrine bladder cancer was diagnosed in the population. Histological grade and T-stage were classified according to the WHO 1973 and 2004 and the 2009 TNM classification, respectively. Serum samples of 97 healthy individuals with no history of cancer were used as controls.

The study was performed in accordance with the ethical standards of the Helsinki Declaration and was approved by the ethical board of the hospital. Samples were centrifuged at 1500 rpm for 15 minutes, immediately aliquoted and kept at -80°C until analysis.
Patient's clinical characteristics are presented in Table 1.

The endpoints of this study were overall- and cancer specific- survival. Cause of death was obtained from death certificates. The median follow-up in all patients was 24 months and the median follow-up in survivors was 31 months. Fifty-six of 188 patients have died during the follow-up period, 39 of them BCa-related.

### Detection of Chromogranin A

Chromogranin A levels were measured on the fully automated B.R.A.H.M.S KRYPTOR® instrument (Thermo Scientific B.R.A.H.M.S GmbH, Hennigsdorf/Berlin, Germany) using an advanced homogeneous sandwich fluoroimmuno-assay commercialized under the reference CgA II #839.050 (patent application WO 2015/158701 A1).

The system uses the sensitive Time Resolved Amplified Cryptate Emission (TRACE®) technology based on a non-radiative energy transfer between a donor (europium cryptate) and an acceptor (XL665). The assay uses two mouse monoclonal antibodies. The epitope of the LK2H10 antibody is localized between amino acid sequence 280-301 and the epitope of the monoclonal antibody PHE5 is localized between amino-acids 124-144. The inter-assay coefficient of variation (CV) is ≤ 12 % and the intra-assay CV of the Kryptor assay is ≤ 7 %. The functional assay sensitivity is 13.7 ng/mL and the quantification limit is 13.71 ng/mL.

### Detection of MMP7

MMP-7 levels were measured on the fully automated B.R.A.H.M.S KRYPTOR® instrument (Thermo Scientific B.R.A.H.M.S GmbH, Hennigsdorf/Berlin, Germany) using an advanced homogeneous sandwich prototype fluoroimmuno-assay (not yet commercialized).

The system uses the sensitive Time Resolved Amplified Cryptate Emission (TRACE®) technology based on a non-radiative energy transfer between a donor (europium cryptate) and an acceptor (XL665). The assay uses a goat polyclonal and a mouse monoclonal anti-human MMP7 antibody labeled with Europium Cryptate -EuC-(Cis Bio International, Bagnols/Cèze, France) and Alexa Fluor 647 (Molecular Probes - Life technologies, Eugene, USA) as donor, respectively. Epitopes of the polyclonal and monoclonal antibodies are not described. Recombinant MMP-7 (R&D Systems Europe) diluted in newborn calf serum (Trina Bioreactives AG, Nänikon, Switzerland) was used as calibrator. The inter-assay coefficient of variation (CV) is ≤ 11% and the intra-assay CV of the Kryptor assay is ≤ 9%. Functional assay sensitivity is 1.95 ng/ml

### Measurements

Single measurements were automatically performed by incubating 50µL of each patient's sample, with 50 µL of each conjugated Antibody solution at 37 °C during 29 min. In order to evaluate reproducibility and repeatability, 2 and 3 quality controls (QC) samples were measured in duplicates in each run for CgA and MMP7 assay, respectively.

### Statistical analysis

The lack of normal distribution of serum concentration data (controlled by Shapiro-Wilk test) indicated the use of non-parametric two-sided Wilcoxon rank sum test (Mann-Whitney test) for independent group comparisons.
For univariate and multivariate analysis, patients were subdivided into low and high concentration groups regarding their CgA and MMP7 levels. Cut-offs were determined to maximize the prognostic value, it corresponds to 147 ng/mL for CGA (80^{th} percentile) and to 7.75 ng/ml for MMP7(66^{th} percentile). Univariate disease-specific survival analysis was done using both Kaplan-Meier log-rank test and univariate Cox analysis. For multivariate analysis, the Cox proportional hazards regression model was used. In all tests the p-value of at least 0.05 was considered to be significant. All statistical analyses were done with SPSS software package, version 21.0 (Chicago, IL, USA).

### Example 2: Preoperative prognostic value of CgA in population treated with surgery (study of Example 1)

### Comparison of CgA serum levels between tumor and control samples

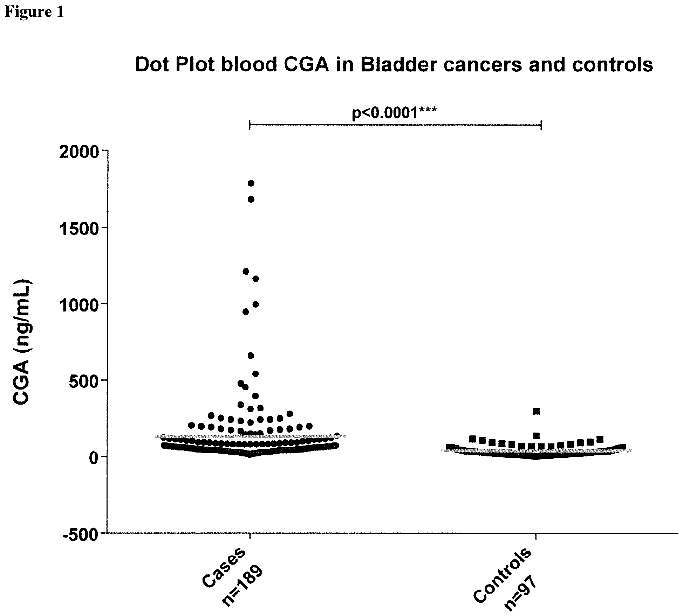

CgA concentrations were significantly elevated in serum samples of tumor patients compared to controls (median 3.9 ng/mL vs 29.4 ng/mL respectively, P < 0.0001, Figure 1).

### CgA concentration and clinicopathological parameters

In tumor patients, CgA concentrations were significantly higher in older patients and in male (p=0.026 and p=0.009 respectively, Table 2) CgA concentrations were correlated to stages and grades but not metastasis (Table 2).

### Prognostic value of CgA levels in population treated with surgery

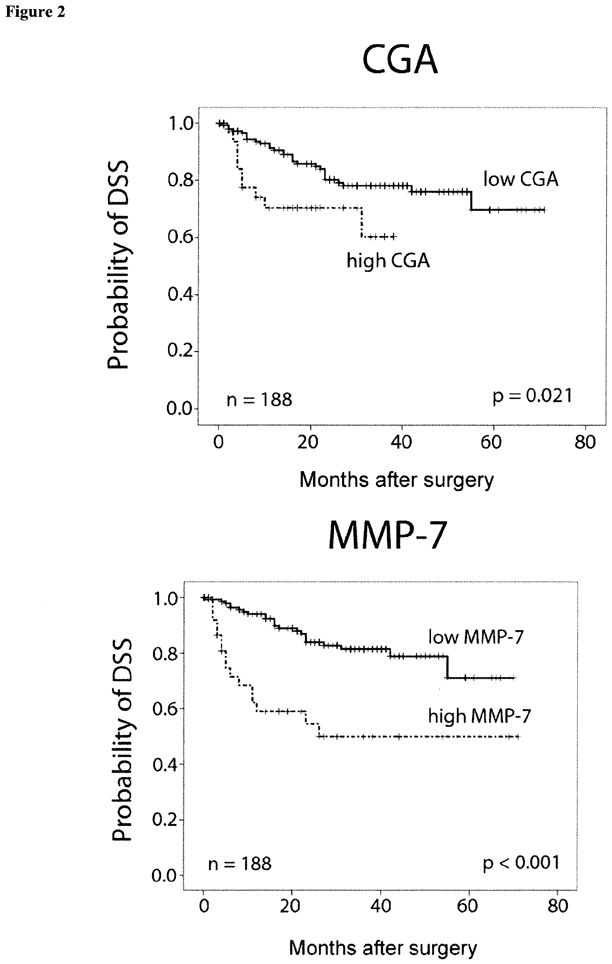

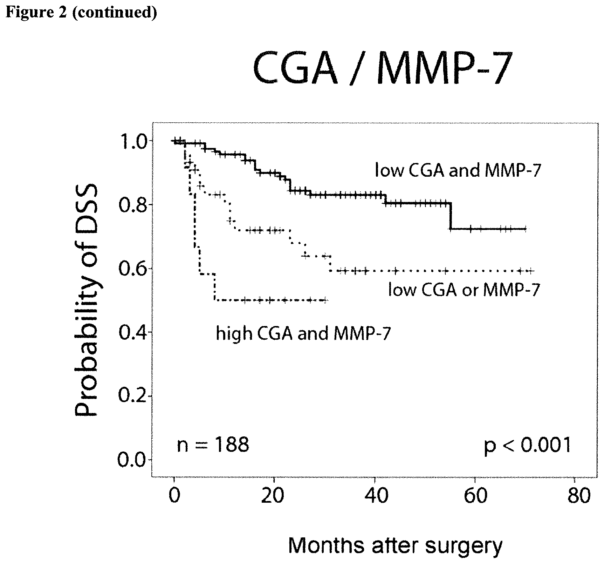

**Univariate analysis:** Results of univariate analysis are listed in Table 3 and Figure 2. Patients' age did not influence overall- or disease-specific survival in contrast to gender that has an impact on disease-specific survival (HR = 0.447, CI 0.247 - 0.918, p = 0.027). Tumor stage, grade and metastasis are significant predictors of overall- and disease-specific survival (P≤0.001, Table 3). CgA serum level is also a strong predictor of overall- and disease-specific survival. High CgA serum concentration was significantly associated with poor overall- and disease-specific survival in patients treated by surgery (HR = 2.553, 95% CI, 1.406-4.566, p=0.002 and HR = 2.295, 95% CI, 1.106 - 4.764, p=0.026, respectively, Table 3; and p =0.021, Figure 2). Furthermore, CgA was also a significant predictor of overall survival when considered as a continuous variable (HR= 1.001, 95% CI, 1.000 - 1.002, p=0.024).

**Multivariate analysis:** Results of multivariate analysis are presented in Table 4. Multivariate analysis demonstrated that high CgA serum concentration is a stage-, grade- and metastasis-independent predictor of overall and disease-specific survival (HR = 3.424, 95% CI, 1.856-6.319, p<0.001 and HR = 3.629; 95% CI, 1.692-7.784; P= 0.001 respectively, Table 4).

Twenty-month-survival rate after treatment was 87% in patients with low preoperative CgA concentration as compared to 70% in patients with a high preoperative CgA level (Figure 2).

Patients with a high preoperative CgA concentration are at high risk of bladder cancer related death and overall mortality and could benefit from more aggressive or experimental treatment, as described in section [0054].

### Preoperative prognostic value of CgA and MMP7 in population treated with surgery

*Comparison of MMP7 serum levels between tumor and control samples:* MMP7 serum concentrations were measured in the same population. MMP7 concentrations were significantly elevated in serum samples of tumor patients compared to controls (median 2.9 ng/mL vs 4.4 ng/mL respectively, P < 0.001).

### MMP7 concentration and clinicopathological parameters

In tumor patients, MMP7 concentrations were significantly higher in older patients (p < 0.001, Table 5) but not in male. MMP7 concentrations were associated with the presence of muscle-invasive tumor and metastasis (p=0.008, p=0.05 respectively, Table 5) but not to grade.

### Prognostic value of MMP7 levels in population treated with surgery

**Univariate analysis:** Results of univariate analysis are presented in Table 3 and Figure 2.
MMP7 serum level is a significant predictor of overall- and disease-specific survival. High MMP7 serum concentration was significantly associated with poor overall- and disease-specific survival in patients treated by surgery (HR = 3.764, 95% CI, 1.983-7.148, p<0.001 and HR = 3.905, 95% CI, 2.291 - 6.655, p<0.001 respectively, Table 3; and p < 0.001, Figure 2).

**Multivariate analysis:** Multivariate analysis demonstrated that high MMP7 serum concentration is a stage-, grade- and metastasis-independent predictor of overall- and disease-specific survival (HR = 2.750, 95% CI 1.557 - 4.855, p<0.001 and HR = 2.324; 95% CI, 1.187 - 4.548; P= 0.014 respectively Table 6).
Twenty-month-survival rate was 89% in patients with low preoperative MMP7 as compared to 59% in patients who had a high preoperative concentration (Figure 2).

### Prognostic value of combined levels of CgA and MMP7 in population treated with surgery

Multivariate analysis demonstrated that high MMP7 and high CgA serum concentrations are stage-, grade- and metastasis-independent predictor of overall- and disease-specific survival (Table 9). High CgA serum concentration combined with high MMP7 concentrations were associated with poor disease-specific survival in patients treated by surgery (p < 0.001, Figure 2).
Twenty-month-survival rate after treatment was over 90% in patients with low preoperative CgA and MMP7, compared to 71% in patients with one elevated serum marker and 50% with both elevated CgA and MMP7 (Figure 2).
Patients with high preoperative CgA and MMP7 concentration are at high risk of bladder cancer related and overall mortality and could benefit from more aggressive or experimental treatment, as described in section [0054].

### Example 3: Preoperative prognostic value of CgA/MMP7 in RCE treated population (study of Example 1)

The risk stratification of patients treated by radical cystectomy is of particular interest. Therefore, we also analyzed the prognostic significance of CgA levels focusing solely on this group.

### Prognostic value of CgA Levels in patients treated with RCE

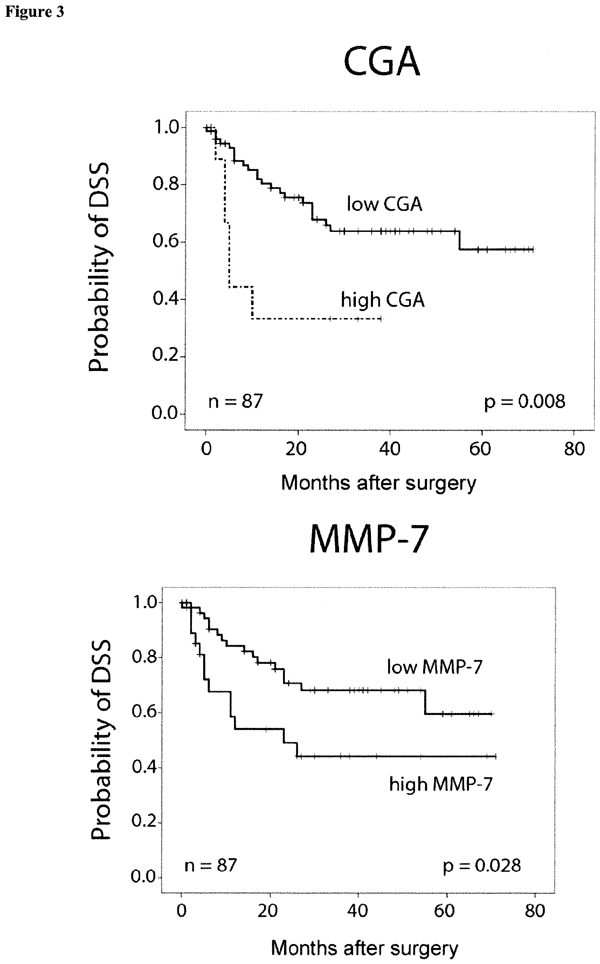

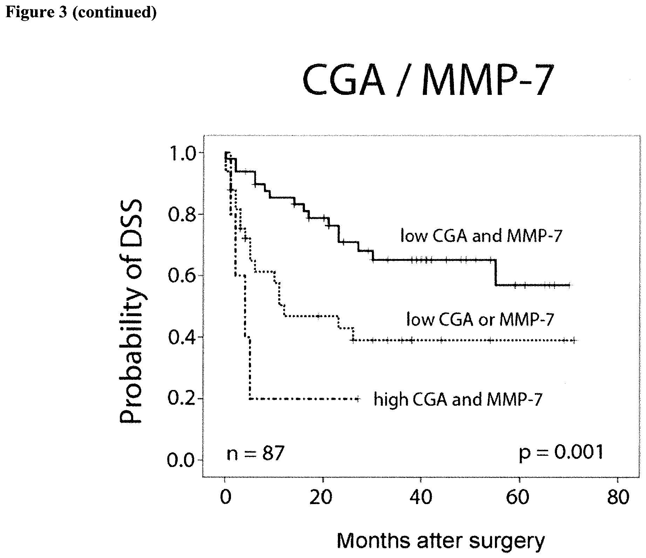

High CgA serum concentration was a strong independent predictor of overall and disease-specific survival (HR = 2.405, 95 CI 1.000 - 5.784, p=0.050 and HR = 4.003, 95% CI 1.491 - 10.748, p=0.006 respectively, Table 7). High CgA concentration was significantly associated with poor disease-specific survival in patients treated by radical cystectomy (p = 0.008, Figure 3).
Twenty-month-survival rate after radical cystectomy was 76% in patients who demonstrated low preoperative concentration of CgA compared to 33% in patients who demonstrated high preoperative concentration.
Patients with high preoperative CgA concentration are at high risk of bladder cancer related and overall mortality and could benefit from more aggressive or experimental treatment, as described in section [0054].

### Prognostic value of MMP7 Levels in patients treated with RCE

High MMP7 serum concentration was a strong independent predictor of overall and disease-specific survival (HR = 2.456, 95% CI 1.294 - 4.662, p=0.006 and HR = 2.195, 95% CI 1.057 - 4.560, p=0.035 respectively, Table 8). High MMP7 concentration was significantly associated to poor disease specific survival in patients treated with radical cystectomy (p = 0.028, Figure 3)

Twenty-month-survival rate after radical cystectomy was 78% in patients who demonstrated low preoperative MMP7 levels compared to 54% in patients who had high preoperative MMP7 concentration (Figure 3).

### Prognostic value of combined levels of CgA and MMP7 in patients treated with RCE

Multivariate analysis demonstrated that high MMP7 and high CgA serum concentrations are stage-, grade- and metastasis-independent predictor of overall- and disease-specific survival (Table 10). High CgA serum concentration combined with high MMP7 concentration were significantly associated with poor disease-specific survival in patients treated by radical cystectomy (p = *0.001*, Figure 3).
Twenty-month-survival rate after radical cystectomy was 79% in patients with low preoperative CgA and MMP7 levels, compared to 47% in patients with one elevated serum marker and 20% with both elevated CgA and MMP7 levels.
Patients with high preoperative CgA and MMP7 concentration are at high risk of bladder cancer related and overall mortality and could benefit from more aggressive or experimental treatment, as described in section [0056].

### Tables

**Table 1 : Patient's clinical characteristics**

| **Variables** | **Cases** | **Controls** |
|---|---|---|
| **Population** | | |
| Size | 188 | 97 |
| Age (median and range) | 71 (21-90) | 63 (52-79) |
| Gender (Male/female) | 149/39 | 56/41 |

| **Classification** | | |
|---|---|---|
| Non-invasive (cis-Ta-T1) | 108 | NA |
| Invasive (T2-T4) | 80 | NA |

| **Stage** | | |
|---|---|---|
| Cis | 8 | NA |
| Ta | 81 | NA |
| T1 | 19 | NA |
| T2 | 28 | NA |
| T3 | 27 | NA |
| T4 | 25 | NA |

| **Grade** | | |
|---|---|---|
| G1 | 37 | NA |
| G2 | 93 | NA |
| G3 | 58 | NA |
| Low-grade(G1-2) | 130 | NA |
| High-g rade(G3) | 58 | NA |

| **Metastases** | | |
|---|---|---|
| No | 156 | NA |
| Yes | 32 | NA |

| **Surgery** | | |
|---|---|---|
| TURB | 101 | NA |
| RCE | 87 | NA |

| | | |
|---|---|---|
| NA : not applicable | | |

**Table 2 : Association of CgA concentration and clinicopathological parameters of tumor population**

| **Variables** | n | **CgA concentration median (ranqe)** | P |
|---|---|---|---|
| **Age** | | | |
| ≤ 65 | 51 | 48.9 (0 - 1682.4) | **0.026** |
| > 65 | 137 | 66.5 (19.4 - 1786.5) | |
| **Gender** | | | |
| Male | 149 | 66.2 (0 - 1786.5) | **0.009** |
| Female | 39 | 45.1 (21.4 - 996.7) | |
| **Stage** | | | |
| Non-inv. (cis-Ta-T1) | 108 | 65.8 (14.6 - 1682.4) | **0.045** |
| Invasive (T2-T4) | 80 | 52.4 (19.4 - 1786.5) | |
| **Grade** | | | |
| Low-grade (G1-2) | 130 | 69.6 (21.2 - 1786.5) | **<0.001** |
| High-grade (G3) | 58 | 46.4 (14.6 - 996.7) | |
| **Metastases** | | | |
| No | 156 | 65.1 (14.6 - 1786.5) | NS |
| Yes | 32 | 47.9 (21.4 - 479.4) | |

**Table 3 : Association of CgA levels, MMP7 levels and clinicopathological parameters with patients' prognosis (univariate analysis)**

| **Variables** | **Overall survival** | | | **Disease-Specific survival** | | |
|---|---|---|---|---|---|---|
| | HR | 95% Cl | P | HR | 95% Cl | P |
| **Age** | | | | | | |
| ≤ 65 | ref. | | | ref. | | |
| > 65 | 1.727 | 0.892 - 3.343 | NS | 1.951 | 0.860 - 4.427 | NS |
| **Gender** | | | | | | |
| Female | ref. | | | ref. | | |
| Male | 0.660 | 0.369 - 1.179 | NS | 0.447 | 0.247 - 0.918 | **0.027** |
| **Stage** | | | | | | |
| Non-inv. (Ta) | ref. | | | ref. | | |
| Invasive (T1-T4) | 3.705 | 2.093 - 6.558 | **<0.001** | 5.449 | 2.583 - | **<0.001** |
| **Grade** | | | | | | |
| Low-grade | ref. | | | ref. | | |
| High-grade | 2.489 | 1.470 - 4.212 | **0.001** | 2.014 | 1.547 - 5.490 | **0.001** |
| **Metastases** | | | | | | |
| No | ref. | | | ref. | | |
| Yes | 5.523 | 3.213 - 9.492 | **<0.001** | 7.125 | 3.757 - | **<0.001** |
| **CgA serum level** | | | | | | |
| Low concentration | ref. | | | ref. | | |
| High concentration | 2.553 | 1.406 - 4.566 | **0.002** | 2.295 | 1.106 - 4.764 | **0.026** |
| **MMP7 serum level** | | | | | | |
| Low concentration | ref. | | | ref. | | |
| High concentration | 3.764 | 1.983 -7.148 | **<0.001** | 3.905 | 2.291 - 6.655 | **<0.001** |

**Table 4 : Association of CgA levels and clinicopathological parameters with Patients' Prognosis (Multivariate analysis)**

| | **Overall survival** | | | **Disease-specific survival** | | |
|---|---|---|---|---|---|---|
| **Variables** | **HR** | **95% CI** | **P** | **HR** | **95% CI** | **P** |
| **All population** | | | | | | |
| Stage (T1-T4) | 1.822 | 0.888 - 3.742 | 0.102 | 3.134 | 1.223 - 8.033 | **0.017** |
| Grade (G3) | 1.184 | 0.595 - 2.356 | 0.630 | 1.074 | 0.479 - 2.410 | 0.862 |
| Metastasis (yes) | 4.129 | 2.026 - 8.414 | **<0.001** | 4.736 | 2.084 - 10.760 | **<0.001** |
| CGA serum (high concentration) | 3.424 | 1.856 - 6.319 | **<0.001** | 3.629 | 1.692 - 7.784 | **0.001** |

**Table 5 : Association of MMP7 concentration with clinicopathological parameters of patients**

| **Variables** | **n** | **MMP7 concentration median (ranqe)** | **P** |
|---|---|---|---|
| **Age** | | | |
| ≤ 65 | 51 | 3.5 (1.6 - 83.4) | **<0.001** |
| > 65 | 137 | 4.9 (1.4 - 72.5) | |
| **Gender** | | | |
| Male | 149 | 4.4 (1.6 - 72.5) | NS |
| Female | 39 | 4.7 (1.4 - 83.4) | |
| **Stage** | | | |
| Non-inv. (cis-Ta-T1) | 108 | 4.1(1.4 - 20.8) | **0.008** |
| Invasive (T2-T4) | 80 | 5.3 (1.6 - 83.4) | |
| **Grade** | | | |
| Low-grade (G1-2) | 130 | 4.4 (1.4 - 28.5) | NS |
| High-grade (G3) | 58 | 4.4 (1.6 - 83.4) | |
| **Metastases** | | | |
| No | 156 | 4.4 (1.4 - 83.4) | **0.05** |
| Yes | 32 | 5.7 (1.6 - 72.5) | |

**Table 6 : Association of MMP7 levels and clinicopathological parameters with patients' prognosis (multivariate analysis)**

| | **Overall survival** | | | **Disease-specific survival** | | |
|---|---|---|---|---|---|---|
| **Variables** | **HR** | **95% CI** | **P** | **HR** | **95% CI** | **P** |
| **All population** | | | | | | |
| Stage (T1-T4) | 2.274 | 1.120 - 4.620 | **0.023** | 3.647 | 1.504 - 8.846 | **0.004** |
| Grade (G3) | 1.405 | 0.758 - 2.603 | 0.280 | 1.349 | 0.657 - 2.768 | 0.415 |
| MMP7 serum (high concentration) | 2.750 | 1.557 - 4.855 | **<0.001** | 2.324 | 1.187 - 4.548 | **0.014** |

**Table 7 : Association of CgA levels and clinicopathological parameters with patient's prognosis in RCE treated population (multivariate analysis)**

| **Variables** | **Overall survival** | | | **Disease-specific survival** | | |
|---|---|---|---|---|---|---|
| | **HR** | **95% Cl** | **P** | **HR** | **95% Cl** | **P** |
| **RCE treated patients** | | | | | | |
| Stage (T1-T4) | 3.508 | 1.466 - 8.397 | **0.005** | 4.581 | 1.623 - 12.932 | **0.004** |
| Grade (G3) | 1.244 | 0.581- 2.666 | 0.574 | 1.107 | 0.464 - 2.6643 | 0.819 |
| Metastasis (yes) | 2.521 | 1.218 - 5.218 | **0.013** | 2.991 | 1.282 - 6.981 | 0.011 |
| CGA serum (high concentration) | 2.405 | 1.000 - 5.784 | **0.050** | 4.003 | 1.491 - 10.748 | **0.006** |

**Table 8 : Association of MMP7 levels and clinicopathological parameters with patient's prognosis in RCE treated population (multivariate analysis**

| **Variables** | **Overall survival** | | | **Disease-specific survival** | | |
|---|---|---|---|---|---|---|
| | **HR** | **95% Cl** | **P** | **HR** | **95% Cl** | **P** |
| **RCE treated patients** | | | | | | |
| Stage (T1-T4) | 3.458 | 1.561- 7.661 | **0.002** | 3.901 | 1.567 - 9.712 | **0.003** |
| Grade (G3) | 1.756 | 0.863 - 3.573 | 0.280 | 1.601 | 0.722 - 3.553 | 0.247 |
| MMP7 serum (high concentration) | 2.456 | 1.294 - 4.662 | **0.006** | 2.195 | 1.057 - 4.560 | **0.035** |

**Table 9: Association of MMP7 and CgA Levels and clinicopathological parameters with patients' prognosis (multivariate analysis)**

| **Variables** | **Overall survival** | | | **Disease-specific survival** | | |
|---|---|---|---|---|---|---|
| | **HR** | **95% Cl** | **P** | **HR** | **95% Cl** | **P** |
| **All population** | | | | | | |
| Stage (T1-T4) | 1466 | 0.698 - 3.083 | 0.313 | 2 668 | 1.020 - 6.980 | **0.046** |
| Grade (G3) | 1391 | 0.704 - 2.749 | 0.343 | 1235 | 0.552 - 2.760 | 0.608 |
| Metastasis (yes) | 3 543 | 1.765 - 7.114 | **<0.001** | 4170 | 1.859 - 9.354 | **0.001** |
| CGA serum (high concentration) | 3164 | 1.681- 5.955 | **<0.001** | 3 485 | 1.598 - 7.603 | **0.012** |
| MMP-7 serum (high concentration) | 2 324 | 1.323 - 4.082 | **0.003** | 1959 | 1.005 - 3.817 | **0.048** |
| Stage (T1-T4) | 1572 | 0.774 - 3.192 | **0.210** | 2 676 | 1.056 - 6.783 | **0.038** |
| Grade (G3) | 1378 | 0.690 - 2.749 | 0.363 | 1324 | 0.576 - 3.046 | 0.509 |
| Metastasis (yes) | 4 035 | 2.003 - 8.131 | **<0.001** | 4 564 | 2.037 - 10.228 | **<0.001** |
| CGA/MMP7 both high | 6 062 | 2.782 - 13.206 | < **0.001** | 7 382 | 2.789 - 19.539 | **<0.001** |

**Table 10 : Association of MMP7 and CgA Levels and clinicopathological parameters with patients' prognosis in RCE treated population (multivariate analysis)**

| **Variables** | **Overall survival** | | | **Disease-specific survival** | | |
|---|---|---|---|---|---|---|
| | **HR** | **95% Cl** | **P** | **HR** | **95% Cl** | **P** |
| **RCE treated patients** | | | | | | |
| Stage (T1-T4) | 3 629 | 1.496 - 8.805 | **0.004** | 4 777 | 1.665 - 13.703 | **0.004** |
| Grade (G3) | 1583 | 0.720 - 3.478 | 0.253 | 1407 | 0.562 - 3.520 | 0.466 |
| Metastasis (yes) | 2 088 | 0.994 - 4.389 | **0.052** | 2 509 | 1.050 - 5.997 | **0.038** |
| CGA serum (high concentration) | 2 713 | 1.110 - 6.634 | **0.029** | 4 556 | 1.652 - 12.563 | **0.003** |
| MMP-7serum(high concentration) | 2149 | 1.090 - 4.237 | **0.027** | 1892 | 0.848 - 4.218 | r 0.119 |
| Stage (T1-T4) | 3 557 | 1.490 - 8.493 | **0.004** | 4 697 | 1.661- 13.286 | **0.004** |
| Grade (G3) | 1462 | 0.670 - 3.191 | 0.340 | 1422 | 0.571- 3.544 | 0.449 |
| Metastasis (yes) | 2 503 | 1.222 - 5.128 | **0.012** | 3 031 | 1.328 - 6.916 | **0.008** |
| CGA/MMP7 both high | 4 878 | 1.539 - 15.463 | **0.007** | 9 059 | 2.170 - 37.820 | **0.003** |

## Claims

1. *In vitro* use of chromogranin A (CgA) as a prognostic marker for bladder cancer, wherein the bladder cancer is non-neuroendocrine bladder cancer.

2. An *in vitro* method for the prognosis, risk assessment, risk stratification, monitoring and/or therapy control of bladder cancer in a subject, comprising the step of determining the level of CgA in a sample of a bodily fluid of said subject, wherein the bladder cancer is non-neuroendocrine bladder cancer.

3. The use according to claim 1 or the method according to claim 2, wherein the bladder cancer is selected from the group consisting of urothelial carcinoma of the bladder, squamous cell carcinoma of the bladder and adenocarcinoma of the bladder,

4. The use according to claim 2 or the method according to claim 3, wherein the bladder cancer is urothelial carcinoma of the bladder.

5. The method according to claim 2 or 3, wherein the level of CgA in the sample from the subject is indicative for the severity of the bladder cancer and/or the outcome for the subject.

6. The method according to claim 5, wherein an increased level of CgA in the sample from the subject as compared to a control level or a predetermined threshold is indicative for a poor outcome for the subject.

7. The method according to claim 5 wherein the level of CgA in the sample from the subject is indicative for the subject's overall survival or the subject's disease-specific survival or the subject's progression-free survival.

8. The method according to claim 6 wherein the poor outcome is an increased risk for a reduced life expectancy, an increased risk of progression, an increased risk of cancer-related death and/or an increased risk of recurrence after surgical treatment and/or drug treatment and/or radiation therapy.

9. The method according to any one of claims 6 to 8 wherein the predetermined threshold for the CgA level is selected in the range of from 100 ng/mL to 431 ng/mL, preferably in the range of from 103 ng/mL to 191 ng/mL, more preferably 130 ng/mL to 160 ng/mL, and most preferably the threshold is 147 ng/mL.

10. The method of any one of claims 2 to 9, wherein additionally the level of matrix metalloproteinase 7 (MMP7) is determined in said sample of said subject, and wherein the level of MMP7 in the sample from the subject is indicative for the severity of the bladder cancer and/or the outcome for the subject.

11. The method according to claims 9 or 10 wherein
(i) a level of CgA in said sample above the predetermined CgA threshold is indicative of a high risk of cancer-related death after surgical treatment and/or drug treatment and/or radiation therapy, and
(ii) a level of CgA in said sample above the predetermined CgA threshold and a level of MMP7 in said sample or another sample of the same subject above the predetermined MMP7 threshold is indicative of very high risk of cancer-related death and shorter lifetime after surgical treatment and/or drug treatment and/or radiation therapy.

12. The method according to any one of claims 6 to 11, wherein the level of CgA and optionally MMP7 is determined at regular time intervals during follow-up, and wherein the control level is the respective level determined before said surgical treatment and/or drug treatment and/or radiation therapy and/or at initial step of the follow-up.

13. The method according to claim 3 wherein the bladder cancer is non-muscular invasive bladder cancer (NMBC) or muscle invasive bladder cancer (MIBC).

14. The method according to any one of claims 2 to 13, wherein the sample is derived from a bodily fluid selected from whole blood, serum, plasma and urine.

15. The method according to any one of claims 2 to 14, wherein the level of CgA is determined using an immunoassay.

## Patentansprüche

1. *In-vitro*-Verwendung von Chromogranin A (CgA) als ein prognostischer Marker für Blasenkrebs, wobei der Blasenkrebs ein nicht-neuroendokriner Blasenkrebs ist.

2. *In-vitro*-Verfahren für die Prognose, Risikobewertung, Risikostratifizierung, Überwachung und/oder Therapiekontrolle von Blasenkrebs in einem Subjekt, umfassend den Schritt eines Bestimmens des CgA-Spiegels in einer Probe einer Körperflüssigkeit des Subjekts, wobei der Blasenkrebs ein nicht-neuroendokriner Blasenkrebs ist.

3. Verwendung nach Anspruch 1 oder Verfahren nach Anspruch 2, wobei der Blasenkrebs aus der Gruppe ausgewählt ist, die aus Urothelkarzinom der Blase, Plattenepithelkarzinom der Blase und Adenokarzinom der Blase besteht.

4. Verwendung nach Anspruch 2 oder Verfahren nach Anspruch 3, wobei der Blasenkrebs ein Urothelkarzinom der Blase ist.

5. Verfahren nach Anspruch 2 oder 3, wobei der CgA-Spiegel in der Probe aus dem Subjekt auf die Schwere des Blasenkrebses und/oder das Ergebnis für das Subjekt hinweist.

6. Verfahren nach Anspruch 5, wobei ein erhöhter CgA-Spiegel in der Probe aus dem Subjekt im Vergleich zu einem Kontrollspiegel oder einem zuvor bestimmten Schwellenwert auf ein schlechtes Ergebnis für das Subjekt hinweist.

7. Verfahren nach Anspruch 5, wobei der CgA-Spiegel in der Probe aus dem Subjekt auf das Gesamtüberleben des Subjekts oder das krankheitsspezifische Überleben des Subjekts oder das progressionsfreie Überleben des Subjekts hinweist.

8. Verfahren nach Anspruch 6, wobei das schlechte Ergebnis ein erhöhtes Risiko für eine geringere Lebenserwartung, ein erhöhtes Risiko einer Progression, ein erhöhtes Risiko eines krebsbedingten Todes und/oder ein erhöhtes Risiko eines Rezidivs nach chirurgischer Behandlung und/oder medikamentöser Behandlung und/oder Strahlentherapie ist.

9. Verfahren nach einem der Ansprüche 6 bis 8, wobei der zuvor bestimmte Schwellenwert für den CgA-Spiegel in dem Bereich von 100 ng/ml bis 431 ng/ml, bevorzugt in dem Bereich von 103 ng/ml bis 191 ng/ml, stärker bevorzugt 130 ng/ml bis 160 ng/ml ausgewählt ist und wobei der Schwellenwert am stärksten bevorzugt 147 ng/ml beträgt.

10. Verfahren nach einem der Ansprüche 2 bis 9, wobei zusätzlich der Matrixmetalloproteinase 7(MMP7)-Spiegel in der Probe aus dem Subjekts bestimmt wird und wobei der MMP7-Spiegel in der Probe aus dem Subjekts auf die Schwere des Blasenkrebses und/oder das Ergebnis für das Subjekt hinweist.

11. Verfahren nach Anspruch 9 oder 10, wobei
(i) ein CgA-Spiegel in der Probe oberhalb des zuvor bestimmten CgA-Schwellenwerts auf ein hohes Risiko eines krebsbedingten Todes nach chirurgischer Behandlung und/oder medikamentöser Behandlung und/oder Strahlentherapie hinweist, und
(ii) ein CgA-Spiegel in der Probe oberhalb des zuvor bestimmten CgA-Schwellenwerts und ein MMP7-Spiegel in der Probe oder einer anderen Probe desselben Subjekts oberhalb des zuvor bestimmten MMP7-Schwellenwerts auf ein sehr hohes Risiko eines krebsbedingten Todes und einer kürzeren Lebensdauer nach chirurgischer Behandlung und/oder medikamentöser Behandlung und/oder Strahlentherapie hinweist.

12. Verfahren nach einem der Ansprüche 6 bis 11, wobei der CgA-Spiegel und optional der MMP7-Spiegel in regelmäßigen Zeitabständen während einer Nachuntersuchung bestimmt wird und wobei der Kontrollspiegel der jeweilige Spiegel ist, der vor der chirurgischen Behandlung und/oder der medikamentösen Behandlung und/oder der Strahlentherapie und/oder bei einem ersten Schritt der Nachuntersuchung bestimmt wird.

13. Verfahren nach Anspruch 3, wobei der Blasenkrebs ein nicht-muskelinvasiver Blasenkrebs *(non-muscularinvasive bladdercancer-* NMBC) oder ein muskelinvasiver Blasenkrebs (*muscle invasive bladder cancer*- MIBC) ist.

14. Verfahren nach einem der Ansprüche 2 bis 13, wobei die Probe von einer Körperflüssigkeit stammt, die aus Vollblut, Serum, Plasma und Urin ausgewählt ist.

15. Verfahren nach einem der Ansprüche 2 bis 14, wobei der CgA-Spiegel unter Verwendung eines Immunoassays bestimmt wird.

## Revendications

1. Utilisation *in vitro* de la chromogranine A (CgA) comme marqueur pronostique du cancer de la vessie, dans laquelle le cancer de la vessie est un cancer de la vessie non neuroendocrinien.

2. Procédé *in vitro* pour le pronostic, l'évaluation de risques, la stratification de risques, la surveillance et/ou la régulation thérapeutique du cancer de la vessie chez un sujet, comprenant l'étape de détermination du niveau de CgA dans un échantillon d'un fluide corporel dudit sujet, dans lequel le cancer de la vessie est un cancer de la vessie non neuroendocrinien.

3. Utilisation selon la revendication 1 ou procédé selon la revendication 2, dans laquelle le cancer de la vessie est choisi dans le groupe constitué par le carcinome urothélial de la vessie, le carcinome spinocellulaire de la vessie et l'adénocarcinome de la vessie.

4. Utilisation selon la revendication 2 ou procédé selon la revendication 3, dans laquelle le cancer de la vessie est un carcinome urothélial de la vessie.

5. Procédé selon la revendication 2 ou 3, dans lequel le niveau de CgA dans l'échantillon du sujet indique la gravité du cancer de la vessie et/ou le résultat pour le sujet.

6. Procédé selon la revendication 5, dans lequel un niveau accru de CgA dans l'échantillon du sujet par rapport à un niveau témoin ou à un seuil prédéterminé indique un mauvais résultat pour le sujet.

7. Procédé selon la revendication 5, dans lequel le niveau de CgA dans l'échantillon du sujet indique la survie globale du sujet ou la survie spécifique à la maladie du sujet ou la survie sans progression du sujet.

8. Procédé selon la revendication 6, dans lequel le mauvais résultat est un risque accru d'espérance de vie réduite, un risque accru de progression, un risque accru de décès lié au cancer et/ou un risque accru de récidive après un traitement chirurgical et/ou un traitement médicamenteux et/ou une radiothérapie.

9. Procédé selon l'une quelconque des revendications 6 à 8, dans lequel le seuil prédéterminé pour le niveau de CgA est choisi dans la plage de 100 ng/mL à 431 ng/mL, de préférence dans la plage de 103 ng/mL à 191 ng/mL, plus préférablement de 130 ng/mL à 160 ng/mL, et idéalement le seuil est de 147 ng/mL.

10. Procédé selon l'une quelconque des revendications 2 à 9, dans lequel en outre le niveau de métalloprotéase matricielle 7 (MMP7) est déterminé dans ledit échantillon dudit sujet, et dans lequel le niveau de MMP7 dans l'échantillon du sujet indique la gravité du cancer de la vessie et/ou du résultat pour le sujet.

11. Procédé selon les revendications 9 ou 10, dans lequel
(i) un niveau de CgA dans ledit échantillon au-dessus du seuil de CgA prédéterminé indique un risque élevé de décès lié au cancer après un traitement chirurgical et/ou un traitement médicamenteux et/ou une radiothérapie, et
(ii) un niveau de CgA dans ledit échantillon au-dessus du seuil de CgA prédéterminé et un niveau de MMP7 dans ledit échantillon ou un autre échantillon du même sujet au-dessus du seuil de MMP7 prédéterminé indique un risque très élevé de décès lié au cancer et une durée de vie plus courte après un traitement chirurgical/ou traitement médicamenteux et/ou une radiothérapie.

12. Procédé selon l'une quelconque des revendications 6 à 11, dans lequel le niveau de CgA et éventuellement de MMP7 est déterminé à des intervalles de temps réguliers pendant le suivi, et dans lequel le niveau témoin est le niveau respectif déterminé avant ledit traitement chirurgical et/ou le traitement médicamenteux et/ou la radiothérapie et/ou à la première étape du suivi.

13. Procédé selon la revendication 3, dans lequel le cancer de la vessie est un cancer de la vessie invasif non musculaire (NMBC) ou un cancer de la vessie invasif musculaire (MIBC).

14. Procédé selon l'une quelconque des revendications 2 à 13, dans lequel l'échantillon est dérivé d'un fluide corporel choisi parmi le sang total, le sérum, le plasma et l'urine.

15. Procédé selon l'une quelconque des revendications 2 à 14, dans lequel le niveau de CgA est déterminé à l'aide d'un immunoessai.
